# EUROPEAN PATENT APPLICATION

(11) **EP 3 928 870 A1**
(43) Date of publication of application: **29.12.2021**
(21) Application number: 20759076.1
(22) Date of filing: 29.01.2020
(51) Int. Cl.: B03C 1/00, B03C 1/01, B03C 1/28, A61M 1/36

(54) **BLOOD PURIFICATION DEVICE AND PURIFICATION METHOD OF BLOOD**

(30) Priority: 22.02.2019 JP 2019030713
(71) Applicant: TDK Corporation, Tokyo 103-6128 (JP)
(72) Inventor: NAGANO Takashi, Tokyo 103-6128 (JP); KITAGAWA Sumiko, Tokyo 103-6128 (JP); KIKUCHI Toshiaki, Tokyo 103-6128 (JP); NOGUCHI Tomohiro, Tokyo 103-6128 (JP)
(74) Representative: Epping - Hermann - Fischer
(86) International application number: PCT/JP2020/003069
(87) International publication number: WO 2020/170735

(57) **Abstract**

Provided is a blood purification device or the like capable of confirming that magnetic particles have been separated and removed from blood.

A blood purification device (1A) includes a main flow channel (20) configured to allow blood (10) flows; a magnetic extraction unit (30) (magnetic extraction means) to collect magnetic particles with a magnetic force, the magnetic particles being contained in blood (10); and one or more magnetic sensor (40) configured to be capable of detecting a presence of the magnetic particles in blood (10). Each of the magnetic particles has, on at least a part of its outer circumferential portion, a modified part being modified with a separation-target capturing material which is capable of capturing a specific substance to be separated in the blood (10).

## Description

### [Technical Field]

The present disclosure relates to a blood purification device and purification method of blood.

Priority is claimed on Japanese Patent Application No. 2019-030713, filed February 22, 2019, the content of which is incorporated herein by reference.

### [Background Art]

A method using centrifugation, membrane separation, an adsorption column separation, or the like is known as a method for removing a causative substance which causes a disease from blood. In addition, a blood purification device which mixes fine particles (magnetic particles), having magnetism and capable of adsorbing a diseasecausing substance in blood thereon, into the blood, and magnetically collects the magnetic particles is known (for example, refer to Patent Literature 1).

### [Citation List]

### [Patent Literature]

### [Patent Literature 1]

Japanese Patent No. 4485380

### [Summary of Invention]

### [Technical Problem]

However, it cannot be said that a blood purification device using magnetic particles in the related art has a sufficient mechanism for confirming that magnetic particles have been removed. Therefore, there is a concern that magnetic particles will remain in blood after purification.

For this reason, there is a problem in that blood after purification cannot be safely returned into the body.

Therefore, an object of the present disclosure is to provide a blood purification device capable of confirming that magnetic particles have been separated and removed from blood, and a purification method of blood.

### [Solution to Problem]

The technology according to the present disclosure includes the following aspects.
[1] A blood purification device including: a main flow channel configured to allow blood to flow through; a magnetic extraction unit (a magnetic extraction means) configured to collect magnetic particles with a magnetic force, the magnetic particles being contained in blood; and one or more magnetic sensors configured to be capable of detecting a presence of the magnetic particles in blood, wherein each of the magnetic particles has, on at least a part of its outer circumferential portion, a modified part being modified with a separation-target capturing material which is capable of capturing a specific substance to be separated in the blood, and wherein the magnetic extraction unit is further configured to collect the magnetic particles that have captured the specific substance and, to extract the collected specific substance from the blood thereby purifying the blood.
[2] The blood purification device according to [1], in which the at least one magnetic sensor among the one or more magnetic sensors is disposed on a subsequent portion with respect to the magnetic extraction unit.
[3] The blood purification device according to [2], in which another at least one magnetic sensor among the one or more magnetic sensors is disposed on a preceding portion with respect to the magnetic extraction unit.
[4] The blood purification device according to any one of [1] to [3], further including: a first flow channel selection unit (a first flow channel selection means) disposed on a preceding portion with respect to the magnetic extraction unit in the main flow channel and configured to select a flow destination of the blood; a second flow channel selection unit (a second flow channel means) disposed on a subsequent portion with respect to the magnetic extraction unit in the main flow channel and configured to select a flow destination of the blood; and a return flow channel disposed between the first flow channel selection unit and the second flow channel selection unit.
[5] The blood purification device according to [4], further including: a magnetic sensor disposed on a subsequent portion with respect to the second flow channel selection unit in the main flow channel.
[6] The blood purification device according to [4] or [5], further including:
   a controller connected to the magnetic sensors, the first flow channel selection unit, and the second flow channel selection unit, wherein the controller is configured to control at least one of the first flow channel selection unit and the second flow channel selection unit on a basis of outputs of the magnetic sensors.
[7] The blood purification device according to any one of [1] to [6], in which the magnetic sensors are magnetoresistive elements which are disposed outside the flow channel so as not to contact with the blood.
[8] The blood purification device according to any one of [1] to [6], in which the magnetic sensors are coil-type sensors which are disposed outside the flow channel so as not to contact with the blood.
[9] The blood purification device according to any one of [1] to [6], in which the magnetic sensors are Hall sensors which are disposed outside the flow channel so as not to contact with the blood.
[10] The blood purification device according to any one of [1] to [9], in which the magnetic extraction unit comprises a magnetic field generator (a magnetic field generation means) configured to generate a magnetic field for collecting the magnetic particles, and wherein the magnetic field generator is disposed at a position outside the flow channel so as not to contact with the blood.
[11] The blood purification device according to any one of [1] to [10], in which a temperature of the blood purification device itself is kept constant or within a specific temperature range.
[12] The blood purification device according to any one of [1] to [11], further including: a validation unit which determines whether the magnetic sensors are normally operational.
[13] A purification method of blood including the steps of: flowing blood containing magnetic particles through a main flow channel being configured to allow the blood to flow through, each of the magnetic particles being modified with a separation-target capturing material which is capable of capturing a specific substance to be separated in blood; collecting at least a portion of the magnetic particles contained in the blood using magnetic extraction unit capable of generating a magnetic field; and detecting, by a magnetic sensor, remaining of the magnetic particles in the blood after the at least the portion of the magnetic particles being collected by the magnetic extraction unit, the magnetic sensor capable of detecting the presence of the magnetic particles.

### [Advantageous Effects of Invention]

According to the present disclosure, it is possible to provide technology for confirming that magnetic particles have been separated and removed from blood.

### [Brief Description of Drawings]

Fig. 1A is a schematic diagram schematically showing a configuration of a plan view of a blood purification device 1A according to a first embodiment of the present disclosure.
Fig. 1B is a schematic diagram schematically showing another configuration of the blood purification device 1A according to the first embodiment of the present disclosure.
Fig. 2A is a schematic diagram schematically showing a configuration of a plan view of a blood purification device 1B according to a second embodiment of the present disclosure.
Fig. 2B is a schematic diagram schematically showing another configuration of the blood purification device 1B according to the second embodiment of the present disclosure.
Fig. 3A is a schematic diagram schematically showing a configuration of a plan view of a blood purification device 1C according to a third embodiment of the present disclosure.
Fig. 3B is a schematic diagram schematically showing another configuration of the blood purification device 1C according to the third embodiment of the present disclosure.
Fig. 4A is a schematic diagram schematically showing a configuration of a plan view of a blood purification device 1D according to a fourth embodiment of the present disclosure.
Fig. 4B is a schematic diagram schematically showing another configuration of the blood purification device ID according to the fourth embodiment of the present disclosure.
Fig. 5A is a schematic diagram schematically showing a configuration of a plan view of a blood purification device IE according to a fifth embodiment of the present disclosure.
Fig. 5B is a schematic diagram schematically showing another configuration of the blood purification device IE according to the fifth embodiment of the present disclosure.
Fig. 6A is a schematic diagram schematically showing a configuration of a plan view of a blood purification device 1F according to a sixth embodiment of the present disclosure.
Fig. 6B is a schematic diagram schematically showing another configuration of the blood purification device IF according to the sixth embodiment of the present disclosure.

### [Description of Embodiments]

Hereinafter, embodiments in which the technology according to the present disclosure can be realized will be described in detail with reference to the drawings. In the drawings used in the following description, a part that becomes a feature is sometimes enlarged for convenience, and the dimensional ratios of each constituent element or the like are not limited to those shown in the drawings.

### [Blood Purification Device]

### (First Embodiment)

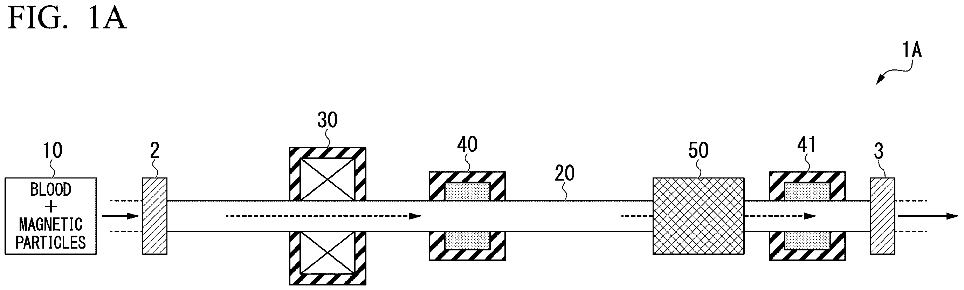

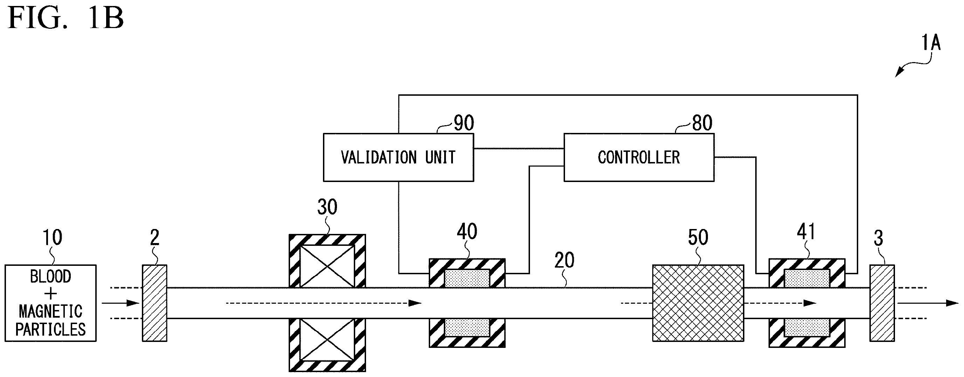

Figs. 1A and 1B are plan views showing configurations of a blood purification device 1A according to a first embodiment of the present disclosure. In the present embodiment, a case where a flow channel through which blood flows does not include a return flow channel will be described as an example.

As exemplified in Fig. 1A, the blood purification device 1A includes: a main flow channel 20 through which blood 10 flows; a magnetic extraction unit 30 (magnetic extraction means) for collecting magnetic particles contained in the blood 10; and magnetic sensors 40 and 41 for detecting the presence of the magnetic particles in the blood 10.

In addition, the blood purification device 1A includes a filter 50, a connection port (inlet) 2, and a connection port (outlet) 3.

The magnetic extraction unit 30, the magnetic sensors 40 and 41 are disposed so as to come into contact with the main flow channel 20. The magnetic sensors 40 and 41 are implemented as parts subsequent to the magnetic extraction unit 30. The number of magnetic sensors implemented as parts subsequent to the magnetic extraction unit 30 in the blood purification device 1A is two, but may be one or may be three or more.

The blood 10 containing magnetic particles flows from the connection port 2 into the main flow channel 20. After the magnetic particles contained in the blood 10 are collected by the magnetic extraction unit 30, the magnetism of the blood 10 is measured by the magnetic sensor 40. As a result, it is determined whether or not the blood 10 contains the magnetic particles. Subsequently, the blood 10 passes through the filter 50, and the magnetic sensor 41 determines whether or not the blood 10 contains the magnetic particles. Thereafter, the blood 10 which has passed through the filter 50 flows out of the connection port 3.

The filter 50 can capture, for example, the magnetic particles which are contained in the blood 10 and could not be removed by the magnetic extraction unit 30, and foreign substances contained in the blood 10.

### <Magnetic Extraction Unit>

The magnetic extraction unit 30 can collect magnetic particles in blood flowing through the main flow channel 20 with a magnetic force. Accordingly, a specific substance captured by the magnetic particles can be extracted from blood.

Known methods can be employed to implement the magnetic extraction unit 30. For example, magnetic separation method disclosed in Patent Literature 1 can be used.

The collection of magnetic particles from blood may be performed, for example, by taking out the magnetic particles into a flow channel connected to the main flow channel 20, or magnetic particles may be collected in a collection unit located on an inner wall of the main flow channel 20.

The magnetic extraction unit 30 may have magnetic field generator (not shown in the drawings) for generating a magnetic field. Magnetic particles are collected from the blood 10 flowing through the main flow channel 20 due to this magnetic field. The magnetic field generator may be, for example, a permanent magnet or a variable magnetic flux magnet, or may be, for example, an induced magnetic field.

The magnetic extraction unit 30 may have a member, such as a magnetic shield layer, capable of shielding a magnetic field. Accordingly, it is possible to suppress or prevent a leakage magnetic field from the magnetic extraction unit 30.

The magnetic field generator may be disposed outside the main flow channel 20 and free of contact with the blood 10. Since the magnetic field generator does not come into contact with the blood 10, coagulation of the blood 10 and contamination with foreign substances can be suppressed.

### <Magnetic Sensor>

The magnetic sensors 40 and 41 can detect the presence of magnetic particles by detecting a leakage magnetic field generated from magnetic particles in blood flowing through the main flow channel 20.

The magnetic sensors 40 and 41 may be disposed outside the main flow channel 20 and free of contact with the blood 10. Since the magnetic sensors 40 and 41 are free of contact with the blood 10, coagulation of the blood 10 and contamination with foreign substances can be suppressed.

Known magnetic sensors can be used as the magnetic sensors 40 and 41. For example, coil-type sensors may be used, Hall sensors may be used, or elements using magnetic resistance may be used. It is possible to determine whether or not the blood 10 flowing through the main flow channel 20 contains magnetic particles based on measurement values of magnetism indicated by the magnetic sensors 40 and 41.

A coil-type sensor measures and detects a magnetic field of magnetic particles which are passing through using an induction coil, when a magnetic particle-containing sample is passed through the induction coil. The coil-type sensor has an advantage of being able to be easily installed.

A Hall sensor detects a magnetic field using the Hall effect and has advantages of being inexpensive and able to be easily installed.

A magnetoresistive element is an element utilizing a phenomenon in which electrical resistance changes due to an effect of a magnetic field, and includes an anisotropic magnetoresistive effect (AMR) element, a giant magnetoresistive (GMR) element, a tunnel magnetoresistive resistance (TMR) element, and the like. A GMR element or a TMR element has advantages that the temperature change or the change with time is small and the sensitivity (MR ratio) is large.

The magnetic sensors 40 and 41 may have a member, such as a magnetic shield layer, capable of shielding a magnetic field. Accordingly, it is possible to prevent failures such as false detection due to influence of a magnetic field generated from the magnetic extraction unit 30.

In a case where it is determined from measurement values of the magnetic sensor 40 and/or the magnetic sensor 41 that the blood 10 does not contain magnetic particles, blood flowing out of the connection port 3 can be safely returned to the body.

In addition, whether or not the blood 10 after passing through the filter 50 contains magnetic particles can be determined based on the measurement values of the magnetic sensor 41, and the blood can be safely returned to the body.

In this case, whether or not magnetic particles are contained in the blood 10 at an amount harmful to the human body may be determined by comparing the measurement values of the magnetic sensors 40 and 41 and a specific reference value (for example, a reference value relating to safety to the human body).

In addition, a signal for controlling an artificial heart-lung machine to be described below may be output from the magnetic sensor 41. According to this configuration, it is possible to confirm that magnetic particles have been sufficiently separated and removed from blood before purified blood is returned to the body, for example.

### <Blood Pump>

The blood purification device 1A may include a blood pump (not shown in the drawings) used for taking out blood from the body. Alternatively, an externally provided blood pump may be used separately from the blood purification device of the present disclosure.

In this blood purification device 1A, for example, the blood 10 containing magnetic particles modified with a separation-target capturing material, which is capable of capturing a component to be separated out, is allowed to flow through the main flow channel 20 through which the blood 10 can flow. In addition, the magnetic extraction unit 30 capable of generating a magnetic field collects at least the portion of the magnetic particles contained in the blood 10. The magnetic sensors 40 and 41 capable of detecting the presence of the magnetic particles detect the presence or the absence of the magnetic particles in the blood after at least the portion of the magnetic particles are collected by the magnetic extraction unit 30.

Although the first embodiment of the present disclosure has been described in detail above, the technology according to the present disclosure is not limited to the first embodiment and various modifications and changes can be made within the scope of the gist of the present disclosure disclosed in the claims.

For example, the temperature of the entire blood purification device 1A in the first embodiment may be kept constant or within a specific temperature range. For example, an exterior cover which externally covers the entire blood purification device 1A and is composed of a heat insulating material or the like may be provided in the blood purification device. For example, the measurement values of the magnetic sensors may be stabilized by keeping the temperature of the entire device constant. In addition, the temperature of the entire blood purification device is preferably kept the same as body temperature. For example, the blood purification device may further include: a temperature sensor (not shown in the drawings) that detects the internal temperature of the above-described exterior cover; and a heating unit (not shown in the drawings) of a heater or the like that heats the interior of the above-described exterior cover according to signals from the controller based on the temperature sensor. Accordingly, it is possible to stabilize the measurement values of the magnetic sensors and to maintain the quality of blood flowing through the blood purification device for a long period of time.

In addition, as exemplified in Fig. 1B, the blood purification device 1A may further include a validation unit 90 which determines whether or not the magnetic sensors 40 and 41 are operating correctly. For example, the validation unit 90 may be connected to the magnetic sensors 40 and 41 and determine whether the magnetic sensors 40 and 41 are operating correctly on a basis of the outputs (magnetic values of blood) from the magnetic sensors. At this time, the validation unit 90 may determine the operations of the magnetic sensors 40 and 41 by, for example, comparing the outputs from the magnetic sensors 40 and 41 with a specific reference value. As an example, the validation unit 90 may determine that the magnetic sensors 40 and 41 are operating correctly in a case where the outputs from the magnetic sensors 40 and 41 are included in a specific range. As another example, the validation unit 90 may determine that the magnetic sensors 40, 41 are not correctly operating in a case where the outputs from the magnetic sensors 40, 41 are greater than a specific upper limit value or less than a specific lower limit value.

The validation unit 90 may be realized, for example, by a circuit element capable of comparing outputs from magnetic sensors with a specific reference value, or by a combination of a general-purpose arithmetic logic device (microprocessor) and an appropriate software program. The configuration of the validation unit 90 is not limited to the above.

The blood purification device 1A may further include a controller 80 (for example, Fig. 1B) which is connected to the magnetic sensors 40 and 41 and determines whether or not magnetic particles are contained in the blood 10 on a basis of the outputs from the magnetic sensors. The controller 80 may check operation states of the magnetic sensors 40 and 41 which have been determined by the validation unit 90. Accordingly, the controller 80 which is a safety circuit can check the operation states of the magnetic sensors 40 and 41. As a result, the controller 80 can accurately determine whether or not magnetic particles are contained in the blood 10.

The controller 80 may be realized, for example, by a circuit element capable of comparing output values from one or more magnetic sensors with each other or output values from magnetic sensors with a specific reference value or the like, or by a combination of a general-purpose arithmetic logic device (microprocessor) and an appropriate software program. The configuration of the controller is not limited to the above.

The validation unit 90 and the controller 80 may be individually realized using a plurality of devices, but may also be integrated as one device.

<Magnetic Particles>

Examples of magnetic particles include particles exhibiting ferromagnetism or paramagnetism. At least a part of the outer circumferential portion of each magnetic particle is modified with a separation-target capturing material which is capable of capturing a specific substance in blood.

The magnetic particles may include: for example, iron, cobalt, nickel, and inorganic compounds thereof; and organically modified inorganic compounds obtained so that these metals or inorganic compounds are modified with organic compounds.

The magnetic particles can capture a specific substance in blood through the separation-target capturing material. A method of capturing a specific component in blood by the separation-target capturing material is not particularly limited. For example, a chemical bonding (such as a molecular biological bonding, an electrical bonding (a van der Waals force, polar attraction, intermolecular forces, and Coulomb force, and the like)), adsorption, and trapping using a three-dimensional structure can be appropriately adopted. Magnetic particles may be coated with coating materials including the separation-target capturing material. Also, whole body magnetic particle may be formed from the separation-target capturing material itself. In addition, magnetic particles may be embedded in the separation-target capturing material being formed in a granular shape.

At least a part of the outer circumferential portion of a magnetic particle is preferably coated with a polymer or a silica matrix depending on the component to be separated out.

At least a part of the outer circumferential portion of a magnetic particle may have hydrophobicity or hydrophilicity depending on the component to be separated out.

The average particle diameter of magnetic particles may be greater than or equal to 2 nm. In a case where the average particle diameter of magnetic particles is within the above-described range, the magnetic particles can be efficiently collected by the magnetic field generator included in the magnetic extraction unit 30. In a case where the average particle diameter thereof is greater than or equal to 100 nm, the effect becomes larger, and in a case where the average particle diameter thereof is greater than or equal to 250 nm, the effect becomes even larger.

The average particle diameter of magnetic particles may be less than or equal to 1 mm. In a case where the average particle diameter of magnetic particles is within the above-described range, the magnetic particles have high dispersibility and a large specific surface area. As a result, it is possible to efficiently capture (for example, adsorb) a specific substance in blood. In a case where the average particle diameter thereof is less than or equal to 10 µm, the effect becomes larger, and in a case where the average particle diameter thereof is less than or equal to 5 µm, the effect becomes even larger.

In the present specification, the "average particle diameter" means a particle diameter (also referred to as a median diameter D₅₀) at 50% of cumulative values in a particle size distribution obtained through a laser diffraction/light scattering method.

Animals from which blood to be purified by the blood purification device 1A is derived are not particularly limited. The blood may be derived from humans or non-human animals such as livestock or pets. Examples of non-human animals include dogs, cats, monkeys, pigs, cows, horses, sheep, goats, mice, rats, and birds.

As described above, the blood purification device 1A can extract a specific substance from blood to purify the blood. The blood purification device 1A may be directly connected to an animal to immediately purify blood collected from the animal. Alternatively, the blood purification device 1A can purify blood which has been collected from an animal in advance without being directly connected to the animal. The purified blood may be immediately returned to the individual animal or may be returned to the individual animal after being stored temporarily.

In the present embodiment, examples of specific substances in blood to be removed by magnetic particles include low-molecular-weight compounds, proteins, nucleic acids, and cells. More specific examples thereof include urea, creatinine, uric acid, β2-micro globulin, LDL cholesterol, abnormal antibodies in immune diseases, and substances, such as viruses, bacteria, fungi, and cancer cells, which cause diseases.

### (Second Embodiment)

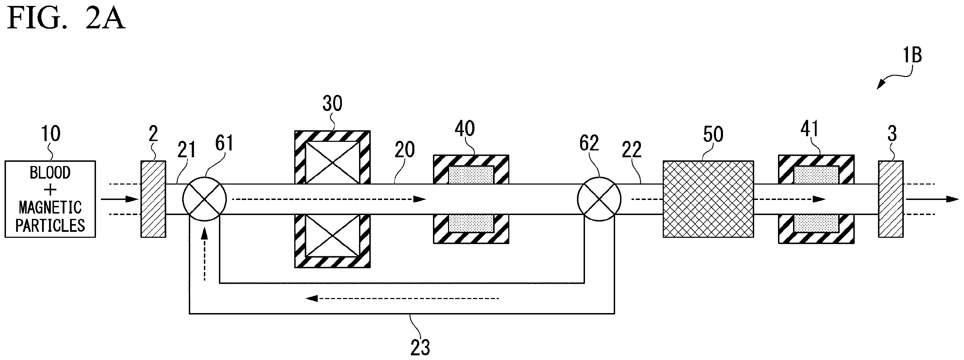

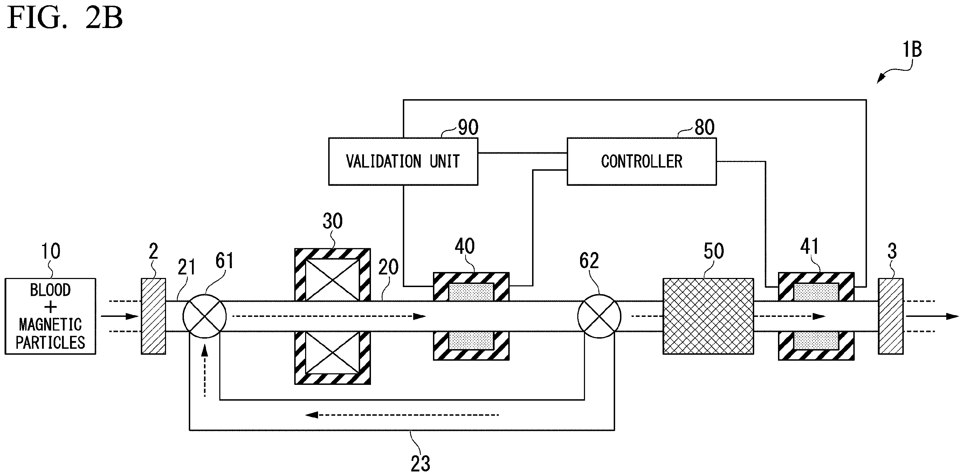

Figs. 2A and 2B are plan views showing configurations of a blood purification device 1B according to a second embodiment of the present disclosure. In the present embodiment, a case where a flow channel through which blood flows includes a return flow channel will be described as an example.

As exemplified in Fig. 2A, the blood purification device 1B includes: main flow channels 20, 21, and 22 through which blood 10 taken out of the body flows; a magnetic extraction unit 30 (magnetic extraction means) for collecting magnetic particles contained in the blood 10; and magnetic sensors 40 and 41 for detecting the presence of the magnetic particles in the blood 10. In addition, the blood purification device 1B includes: a return flow channel 23 for recirculating the blood 10; a first flow channel selection unit 61 (first flow channel selection means) for selecting a flow destination of blood; and a second flow channel selection unit 62 (second flow channel selection means) for selecting a flow destination of blood. Furthermore, the blood purification device 1B includes a filter 50, a connection port (inlet) 2, and a connection port (outlet) 3.

The magnetic extraction unit 30 and the magnetic sensor 40 are disposed so as to come into contact with the main flow channel 20, and the magnetic sensor 41 is disposed so as to come into contact with the main flow channel 22. The magnetic sensors 40 and 41 are implemented as parts subsequent to the magnetic extraction unit 30. In a embodiment of in the blood purification device 1B, two magnetic sensors are implemented as parts subsequent to the magnetic extraction unit 30, however the number of magnetic sensor may be one or may be three or more.

In the main flow channel 20, the first flow channel selection unit 61 is disposed on a preceding portion with respect to the magnetic extraction unit 30, and the second flow channel selection unit 62 is disposed on a subsequent portion with respect to the magnetic extraction unit 30. In the blood purification device 1B, the first flow channel selection unit 61 and the second flow channel selection unit 62 may be operated manually, for example. As another example, the first flow channel selection unit 61 (flow channel selection means) and the second flow channel selection unit 62 (flow channel selection means) may be controlled using an appropriate actuator or the like. The first flow channel selection unit 61 and the second flow channel selection unit 62 are, for example, three-way valves.

First, the first flow channel selection unit 61 is operated to allow communication between the main flow channel 21 and the main flow channel 20, and the second flow channel selection unit 62 is operated to allow communication between the main flow channel 20 and the return flow channel 23 and block communication between the main flow channels 20 and 22. Subsequently, the blood 10 containing magnetic particles flows from the connection port 2 into the main flow channel 21.

After the blood 10 containing magnetic particles flows in from the connection port 2, the first flow channel selection unit 61 is operated to allow communication between the main flow channel 20 and the return flow channel 23. At this time, the second flow channel selection unit 62 allows a state where the main flow channel 20 is allowed to communicate with the return flow channel 23 to continue.

The blood 10 circulates in the main flow channel 20 and the return flow channel 23. During that period, the magnetic particles are collected by the magnetic extraction unit 30, and the magnetism generated from the blood 10 is measured by the magnetic sensor 40.

When it is determined from the measurement value shown by the magnetic sensor 40 that the blood 10 flowing through the main flow channel 20 contains magnetic particles, the first flow channel selection unit 61 and the second flow channel selection unit 62 allow the state where the main flow channel 20 is allowed to communicate with the return flow channel 23 to continue. The blood 10 containing magnetic particles recirculates through the main flow channel 20 and the return flow channel 23, and the magnetic particles contained in the blood 10 are collected by the magnetic extraction unit 30.

When it is determined from the measurement value shown by the magnetic sensor 40 that the blood 10 flowing through the main flow channel 20 does not contain magnetic particles, the first flow channel selection unit 61 allows the state where the main flow channel 20 is allowed to communicate with the return flow channel 23 to continue and the second flow channel selection unit 62 is operated to allow communication between the main flow channel 20 and the main flow channel 22.

Then, the blood 10 flows into the main flow channel 22 and passes through the filter 50. After the magnetic sensor 41 determines whether or not the blood 10 contains magnetic particles, the blood 10 flowing through the main flow channel 22 flows out of the connection port 3.

In a case where it is determined from measurement values of the magnetic sensor 40 and/or the magnetic sensor 41 that the blood 10 does not contain magnetic particles, blood flowing out of the connection port 3 can be safely returned to the body. In addition, whether or not the blood 10 after passing through the filter 50 contains magnetic particles can be determined based on the measurement values of the magnetic sensor 41, and the blood can be safely returned to the body.

In addition, a signal for controlling an artificial heart-lung machine to be described below may be output from the magnetic sensor 41. According to this configuration, it is possible to confirm that magnetic particles have been sufficiently separated and removed from blood before purified blood is returned to the body.

The filter 50 can capture, for example, the magnetic particles which are contained in the blood 10 and could not be removed by the magnetic extraction unit 30, and foreign substances contained in the blood 10.

### <Magnetic Extraction Unit>

The magnetic extraction unit 30 can collect magnetic particles in blood flowing through the main flow channel 20 with a magnetic force. Accordingly, a specific substance captured by the magnetic particles can be extracted from blood.

Known methods can be employed to implement the magnetic extraction unit 30. For example, magnetic separation method disclosed in Patent Literature 1 can be used.

The collection of magnetic particles from blood may be performed, for example, by taking out the magnetic particles into a flow channel connected to the main flow channel 20, or magnetic particles may be collected in a collection unit located on an inner wall of the main flow channel 20.

The magnetic extraction unit 30 may have magnetic field generator (not shown in the drawings) for generating a magnetic field. Magnetic particles are collected from the blood 10 flowing through the main flow channel 20 due to this magnetic field. The magnetic field generator may be, for example, a permanent magnet or a variable magnetic flux magnet, or may be, for example, an induced magnetic field.

The magnetic extraction unit 30 may have a member, such as a magnetic shield layer, capable of shielding a magnetic field. Accordingly, it is possible to suppress or prevent a leakage magnetic field from the magnetic extraction unit 30.

The magnetic field generator may be disposed outside the main flow channel 20 and free of contact with the blood 10. Since the magnetic field generator does not come into contact with the blood 10, coagulation of the blood 10 and contamination with foreign substances can be suppressed.

### <Magnetic Sensor>

The magnetic sensor 40 can detect the presence of magnetic particles by detecting a leakage magnetic field generated from magnetic particles in blood flowing through the main flow channel 20. The magnetic sensor 41 can detect the presence of magnetic particles by detecting a leakage magnetic field generated from magnetic particles in blood flowing through the main flow channel 22.

The magnetic sensors 40 and 41 may be respectively disposed outside the main flow channels 20 and 22 and free of contact with the blood 10. Since the magnetic sensors 40 and 41 are free of contact with the blood 10, coagulation of the blood 10 and contamination with foreign substances can be suppressed.

Known magnetic sensors can be used as the magnetic sensors 40 and 41. For example, coil-type sensors may be used, Hall sensors may be used, or elements using magnetic resistance may be used. It is possible to determine whether or not the blood 10 flowing through the main flow channel 20 contains magnetic particles based on measurement values of magnetism indicated by the magnetic sensors 40 and 41.

A coil-type sensor measures and detects a magnetic field of magnetic particles which are passing through using an induction coil, when a magnetic particle-containing sample is passed through the induction coil. The coil-type sensor has an advantage of being able to be easily installed.

A Hall sensor detects a magnetic field using the Hall effect and has advantages of being inexpensive and able to be easily installed.

A magnetoresistive element is an element utilizing a phenomenon in which electrical resistance changes due to an effect of a magnetic field, and includes an anisotropic magnetoresistive effect (AMR) element, a giant magnetoresistive (GMR) element, a tunnel magnetoresistive resistance (TMR) element, and the like. A GMR element or a TMR element has advantages that the temperature change or the change with time is small and the sensitivity (MR ratio) is large.

The magnetic sensors 40 and 41 may have a member, such as a magnetic shield layer, capable of shielding a magnetic field. Accordingly, it is possible to prevent failures such as false detection due to influence of a magnetic field generated from the magnetic extraction unit 30.

### <Blood Pump>

The blood purification device 1B may be connected to a blood pump (not shown in the drawings) capable of making blood flow. The blood pump may be, for example, used for taking out blood from the body or used for circulating blood taken out of the body. Alternatively, an externally provided blood pump may be used separately from the blood purification device of the present disclosure.

In this blood purification device 1B, for example, the blood 10 containing magnetic particles modified with the separation-target capturing material, which is capable of capturing a specific substance in the blood, is allowed to flow through the main flow channels 20, 21, and 22 through which the blood 10 can flow. In addition, the magnetic extraction unit 30 capable of generating a magnetic field collects at least the portion of the magnetic particles contained in the blood 10. The magnetic sensors 40 and 41 capable of detecting the presence of the magnetic particles detect the presence or the absence of the magnetic particles in the blood after at least the portion of the magnetic particles are collected by the magnetic extraction unit 30.

Although the second embodiment of the technology according to the present disclosure has been described in detail above, the technology according to the present disclosure is not limited to the second embodiment and various modifications and changes can be made within the scope of the gist of the present disclosure disclosed in the claims.

For example, the temperature of the entire blood purification device 1B in the second embodiment may be kept constant or within a specific temperature range. For example, an exterior cover which externally covers the entire blood purification device and is composed of a heat insulating material or the like may be provided in the blood purification device. The measurement values of the magnetic sensors are stabilized by keeping the temperature of the entire device constant. In addition, the temperature of the entire blood purification device is preferably kept the same as body temperature. For example, the blood purification device may further include: a temperature sensor (not shown in the drawings) that detects the internal temperature of the above-described exterior cover; and a heating unit (not shown in the drawings) of a heater or the like that heats the interior of the above-described exterior cover according to signals from the controller based on the temperature sensor. Accordingly, it is possible to stabilize the measurement values of the magnetic sensors and to maintain the quality of blood flowing through the blood purification device for a long period of time.

In addition, as exemplified in Fig. 2B, the blood purification device 1B may further include a validation unit 90 which determines whether or not the magnetic sensors 40 and 41 are operating correctly. For example, the validation unit 90 may be connected to the magnetic sensors 40 and 41 and determine whether the magnetic sensors are operating correctly on a basis of the outputs (magnetic values of blood) from the magnetic sensors. The validation unit 90 of this second embodiment may be configured in the same manner as the validation unit 90 of the above-described first embodiment, for example.

In addition, the blood purification device 1B may further include a controller 80 (for example, Fig. 2B) which is connected to the magnetic sensors 40 and 41 and determines whether or not magnetic particles are contained in the blood 10 on a basis of the outputs from the magnetic sensors. The controller 80 may check operation states of the magnetic sensors 40 and 41 which have been determined by the validation unit 90. The controller 80 of this second embodiment can be configured in the same manner as the controller 80 of the above-described first embodiment, for example. Accordingly, the controller 80 which is a safety circuit can check the operations of the magnetic sensors 40 and 41. As a result, the controller can accurately determine whether or not magnetic particles are contained in the blood 10.

### <Magnetic Particles>

Examples of magnetic particles include particles exhibiting ferromagnetism or paramagnetism. At least a part of the outer circumferential portion of each magnetic particle is modified with a separation-target capturing material which is capable of capturing a specific substance in blood.

The magnetic particles may include: for example, iron, cobalt, nickel, and inorganic compounds thereof; and organically modified inorganic compounds obtained so that these metals or inorganic compounds are modified with organic compounds.

The magnetic particles can capture a specific substance in blood through the separation-target capturing material. A method of capturing a specific component in blood by the separation-target capturing material is not particularly limited. For example, a chemical bonding (such as a molecular biological bonding, an electrical bonding (a van der Waals force, polar attraction, intermolecular forces, and Coulomb force, and the like)), adsorption, and trapping using a three-dimensional structure can be appropriately adopted. Magnetic particles may be coated with coating materials including the separation-target capturing material. Also, whole body of magnetic particles may be formed from the separation-target capturing material itself.

At least a part of the outer circumferential portion of a magnetic particle is preferably coated with a polymer or a silica matrix depending on the component to be separated out.

At least a part of the outer circumferential portion of a magnetic particle may have hydrophobicity or hydrophilicity depending on the component to be separated out.

The average particle diameter of magnetic particles may be greater than or equal to 2 nm. In a case where the average particle diameter of magnetic particles is within the above-described range, the magnetic particles can be efficiently collected by the magnetic field generator included in the magnetic extraction unit 30. In a case where the average particle diameter thereof is greater than or equal to 100 nm, the effect becomes larger, and in a case where the average particle diameter thereof is greater than or equal to 250 nm, the effect becomes even larger.

The average particle diameter of magnetic particles may be less than or equal to 1 mm. In a case where the average particle diameter of magnetic particles is within the above-described range, the magnetic particles have high dispersibility and a large specific surface area. As a result, it is possible to efficiently capture (for example, adsorb) a specific substance in blood. In a case where the average particle diameter thereof is less than or equal to 10 µm, the effect becomes larger, and in a case where the average particle diameter thereof is less than or equal to 5 µm, the effect becomes even larger.

In the present specification, the "average particle diameter" means a particle diameter (also referred to as a median diameter D₅₀) at 50% of cumulative values in a particle size distribution obtained through a laser diffraction/light scattering method.

Animals from which blood to be purified by the blood purification device 1B is derived are not particularly limited. The blood may be derived from humans or non-human animals such as livestock or pets. Examples of non-human animals include dogs, cats, monkeys, pigs, cows, horses, sheep, goats, mice, rats, and birds.

As described above, the blood purification device 1B can extract a specific substance from blood to purify the blood. The blood purification device 1B may be directly connected to an animal to immediately purify blood collected from the animal. Alternatively, the blood purification device 1B can purify blood which has been collected from an animal in advance without being directly connected to the animal. The purified blood may be immediately returned to the individual animal or may be returned to the individual animal after being stored temporarily.

In the present embodiment, examples of specific substances in blood to be removed by magnetic particles include low-molecular-weight compounds, proteins, nucleic acids, and cells. More specific examples thereof include urea, creatinine, uric acid, β2-micro globulin, LDL cholesterol, abnormal antibodies in immune diseases, and substances, such as viruses, bacteria, fungi, and cancer cells, which cause diseases.

### (Third Embodiment)

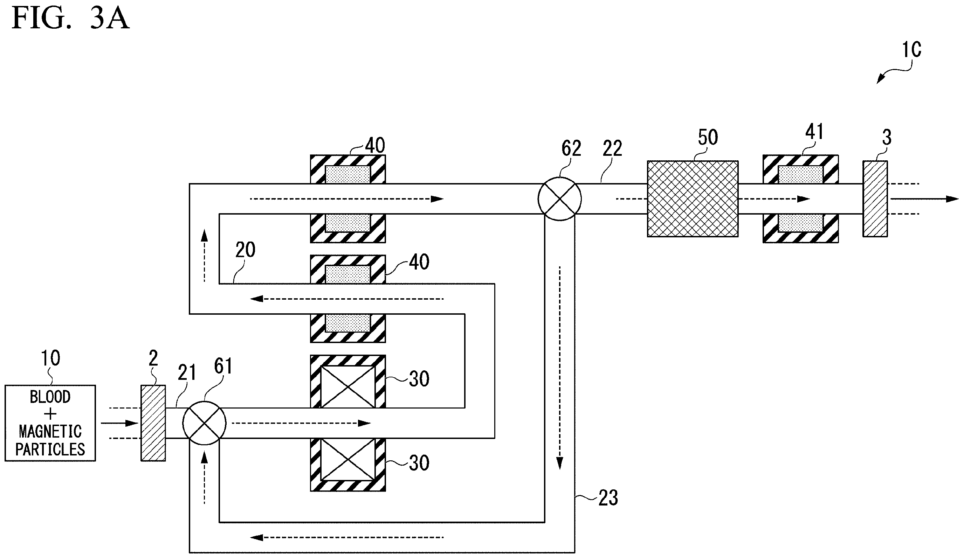

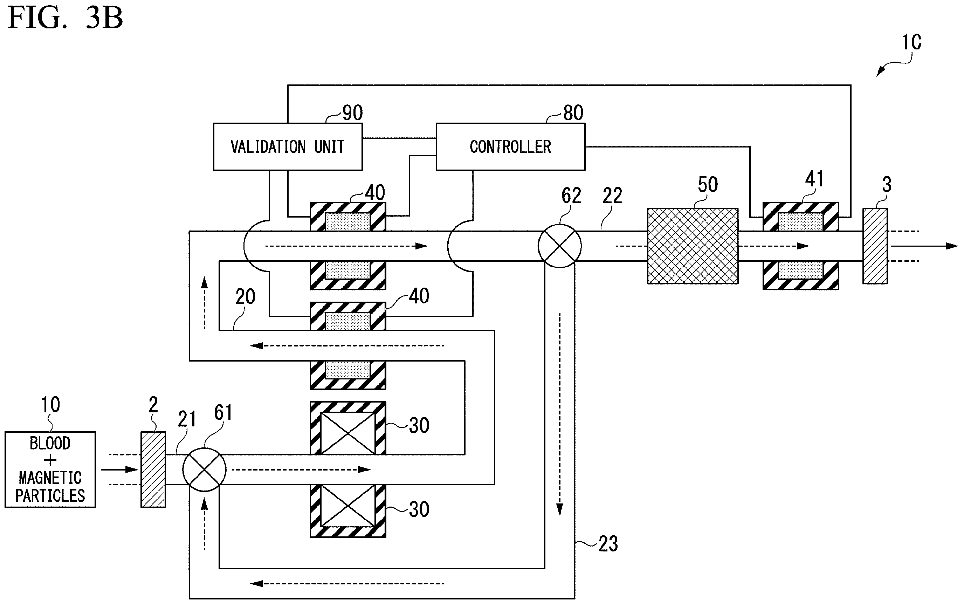

Figs. 3A and 3B are plan views showing configurations of a blood purification device 1C according to a third embodiment of the present disclosure. In the present embodiment, a case where a flow channel through which blood flows includes a meander-shaped return flow channel will be described as an example.

As exemplified in Fig. 3A, the blood purification device 1C includes: main flow channels 20, 21, and 22 through which blood 10 flows; a magnetic extraction unit 30 (magnetic extraction means) for collecting magnetic particles contained in the blood 10; and magnetic sensors 40 and 41 for detecting the presence of the magnetic particles in the blood 10. In addition, the blood purification device 1C includes: a return flow channel 23 for recirculating the blood 10; a first flow channel selection unit 61 (first flow channel selection means) for selecting a flow channel; and a second flow channel selection unit 62 for selecting a flow channel. Furthermore, the blood purification device 1C includes a filter 50, a connection port (inlet) 2, and a connection port (outlet) 3.

The magnetic extraction unit 30 and the two magnetic sensors 40 and 40 are disposed so as to come into contact with the main flow channel 20, and the magnetic sensor 41 is disposed so as to come into contact with the main flow channel 22. The magnetic sensors 40, 40, and 41 are implemented as parts subsequent to the magnetic extraction unit 30. In an embodiment of the blood purification device 1C, three magnetic sensors may be implemented as parts subsequent to the magnetic extraction unit 30, however, the number of the magnetic sensor(s) may be one or two or may be four or more.

In the blood purification device 1C, the two magnetic sensors 40 and 40 implemented in the main flow channel 20 are, for example, attached in a state of being deviated from the axis of the magnetic extraction unit 30 in a side view of the main flow channel 20. Furthermore, the two magnetic sensors 40 and 40 are also attached in a state in which their axes are deviated from each other. In this manner, the two magnetic sensors 40 and 40 are disposed so as not to be affected by a magnetic field generated by the magnetic extraction unit 30.

By providing the two magnetic sensors 40 in this blood purification device 1C, it is possible to more accurately measure the magnetism of the blood 10 using two measurement values of the two magnetic sensors 40 and 40. In addition, with such an arrangement, malfunction hardly occurs in the magnetic sensors 40, and therefore, the measurement accuracy of the magnetic sensors 40 can be improved.

In the main flow channel 20, the first flow channel selection unit 61 is disposed on a preceding portion with respect to the magnetic extraction unit 30, and the second flow channel selection unit 62 is disposed on a subsequent portion with respect to the magnetic extraction unit 30. In the blood purification device 1C, the first flow channel selection unit 61 and the second flow channel selection unit 62 may be operated manually, for example. As another example, the first flow channel selection unit 61 and the second flow channel selection unit 62 may be controlled using an appropriate actuator or the like. The first flow channel selection unit 61 and the second flow channel selection unit 62 are, for example, three-way valves.

First, the first flow channel selection unit 61 is operated to allow communication between the main flow channel 21 and the main flow channel 20, and the second flow channel selection unit 62 is operated to allow communication between the main flow channel 20 and the return flow channel 23. Subsequently, the blood 10 containing magnetic particles flows from the connection port 2 into the main flow channel 21.

After the blood 10 containing magnetic particles flows in from the connection port 2, the first flow channel selection unit 61 is operated to allow communication between the main flow channel 20 and the return flow channel 23. At this time, the second flow channel selection unit 62 allows a state where the main flow channel 20 is allowed to communicate with the return flow channel 23 to continue.

The blood 10 circulates in the main flow channel 20 and the return flow channel 23. During that period, the magnetic particles are collected by the magnetic extraction unit 30, and the magnetism generated from the blood 10 is measured by the magnetic sensors 40.

When it is determined from the measurement values shown by the magnetic sensors 40 that the blood 10 flowing through the main flow channel 20 contains magnetic particles, the first flow channel selection unit 61 and the second flow channel selection unit 62 allow the state where the main flow channel 20 is allowed to communicate with the return flow channel 23 to continue. The blood 10 containing magnetic particles recirculates through the main flow channel 20 and the return flow channel 23, and the magnetic particles contained in the blood 10 are collected by the magnetic extraction unit 30.

When it is determined from the measurement values shown by the magnetic sensors 40 that the blood 10 flowing through the main flow channel 20 does not contain magnetic particles, the first flow channel selection unit 61 allows the state where the main flow channel 20 is allowed to communicate with the return flow channel 23 to continue and the second flow channel selection unit 62 is operated to allow communication between the main flow channel 20 and the main flow channel 22.

Then, the blood 10 flows into the main flow channel 22 and passes through the filter 50. After the magnetic sensor 41 determines whether or not the blood 10 contains magnetic particles, the blood 10 flowing through the main flow channel 22 flows out of the connection port 3.

In a case where it is determined from measurement values of the magnetic sensors 40 and/or the magnetic sensor 41 that the blood 10 does not contain magnetic particles, blood flowing out of the connection port 3 can be safely returned to the body. In addition, whether or not the blood 10 that has been passed through the filter 50 contains magnetic particles can be determined based on the measurement values of the magnetic sensor 41, and the blood can be safely returned to the body.

In addition, a signal for controlling an artificial heart-lung machine to be described below may be output from the magnetic sensor 41. According to this configuration, it is possible to confirm that magnetic particles have been sufficiently separated and removed from blood before purified blood is returned to the body.

The filter 50 can capture, for example, the magnetic particles which are contained in the blood 10 and could not be removed by the magnetic extraction unit 30, and foreign substances contained in the blood 10.

### <Magnetic Extraction Unit>

The magnetic extraction unit 30 can collect magnetic particles in blood flowing through the main flow channel 20 with a magnetic force. Accordingly, a specific substance captured by the magnetic particles can be extracted from blood.

Known methods can be employed to implement the magnetic extraction unit 30. For example, magnetic separation method disclosed in Patent Literature 1 can be used.

The collection of magnetic particles from blood may be performed, for example, by taking out the magnetic particles into a flow channel connected to the main flow channel 20, or magnetic particles may be collected in a collection unit located on an inner wall of the main flow channel 20.

The magnetic extraction unit 30 may have magnetic field generator (not shown in the drawings) for generating a magnetic field. Magnetic particles are collected from the blood 10 flowing through the main flow channel 20 due to this magnetic field. The magnetic field generator may be, for example, a permanent magnet or a variable magnetic flux magnet, or may be, for example, an induced magnetic field.

The magnetic extraction unit 30 may have a member, such as a magnetic shield layer, capable of shielding a magnetic field. Accordingly, it is possible to suppress or prevent a leakage magnetic field from the magnetic extraction unit 30.

The magnetic field generator may be disposed outside the main flow channel 20 and free of contact with the blood 10. Since the magnetic field generator does not come into contact with the blood 10, coagulation of the blood 10 and contamination with foreign substances can be suppressed.

### <Magnetic Sensor>

The magnetic sensors 40 and 41 can detect the presence of magnetic particles by detecting a leakage magnetic field generated from magnetic particles in blood flowing through the main flow channel 20.

The magnetic sensors 40 and 41 may be respectively disposed outside the main flow channels 20 and 22 and free of contact with the blood 10. Since the magnetic sensors 40 and 41 are free of contact with the blood 10, coagulation of the blood 10 and contamination with foreign substances can be suppressed.

Known magnetic sensors can be used as the magnetic sensors 40 and 41. For example, coil-type sensors may be used, Hall sensors may be used, or elements using magnetic resistance may be used. It is possible to determine whether or not the blood 10 flowing through the main flow channel 20 contains magnetic particles based on measurement values of magnetism indicated by the magnetic sensors 40 and 41.

A coil-type sensor measures and detects a magnetic field of magnetic particles which are passing through using an induction coil, when a magnetic particle-containing sample is passed through the induction coil. The coil-type sensor has an advantage of being able to be easily installed.

A Hall sensor detects a magnetic field using the Hall effect and has advantages of being inexpensive and able to be easily installed.

A magnetoresistive element is an element utilizing a phenomenon in which electrical resistance changes due to an effect of a magnetic field, and includes an anisotropic magnetoresistive effect (AMR) element, a giant magnetoresistive (GMR) element, a tunnel magnetoresistive resistance (TMR) element, and the like. A GMR element or a TMR element has advantages that the temperature change or the change with time is small and the sensitivity (MR ratio) is large.

The magnetic sensors 40 and 41 may have a member, such as a magnetic shield layer, capable of shielding a magnetic field. Accordingly, it is possible to prevent failures such as false detection due to influence of a magnetic field generated from the magnetic extraction unit 30.

### <Blood Pump>

The blood purification device 1C may be connected to a blood pump capable of making blood flow. The blood pump may be, for example, used for taking out blood from the body or used for circulating blood taken out of the body. Alternatively, an externally provided blood pump may be used separately from the blood purification device of the present disclosure.

In this blood purification device 1C, for example, the blood 10 containing magnetic particles modified with the separation-target capturing material, is allowed to flow through the main flow channels 20, 21, and 22 through which the blood 10 can flow. In addition, the magnetic extraction unit 30 capable of generating a magnetic field collects at least the portion of the magnetic particles contained in the blood 10. The magnetic sensors 40 and 41 capable of detecting the presence of the magnetic particles detect the presence or the absence of the magnetic particles in the blood after at least the portion of the magnetic particles are collected by the magnetic extraction unit 30.

Although the third embodiment of the present disclosure has been described in detail above, the technology according to the present disclosure is not limited to the third embodiment and various modifications and changes can be made within the scope of the gist of the present disclosure disclosed in the claims.

For example, the temperature of the entire blood purification device 1C in the third embodiment may be kept constant or within a specific temperature range. For example, an exterior cover which externally covers the entire blood purification device and is composed of a heat insulating material or the like may be provided in the blood purification device. The measurement values of the magnetic sensors are stabilized by keeping the temperature of the entire device constant. In addition, the temperature of the entire blood purification device is preferably kept the same as body temperature. For example, the blood purification device may further include: a temperature sensor (not shown in the drawings) that detects the internal temperature of the above-described exterior cover; and a heating unit (not shown in the drawings) of a heater or the like that heats the interior of the above-described exterior cover according to signals from the controller based on the temperature sensor. Accordingly, it is possible to stabilize the measurement values of the magnetic sensors and to maintain the quality of blood flowing through the blood purification device for a long period of time.

In addition, as exemplified in Fig. 3B, the blood purification device 1C may further include a validation unit 90 which determines whether or not the magnetic sensors 40, 40, and 41 are operating correctly. For example, the validation unit 90 may be connected to the magnetic sensors 40, 40, and 41 and determine whether the magnetic sensors 40, 40, and 41 are operating correctly on a basis of the outputs (magnetic values of blood) from the magnetic sensors. The validation unit may be configured in the same manner as the validation unit 90 of the above-described first embodiment, for example.

In addition, the blood purification device 1C may further include a controller 80 (for example, Fig. 3B) which is connected to the magnetic sensors 40, 40, and 41 and determines whether or not magnetic particles are contained in the blood 10 on a basis of the outputs from the magnetic sensors. The controller 80 may check operation states of the magnetic sensors 40, 40, and 41 which have been determined by the validation unit 90. The controller 80 of this third embodiment can be configured in the same manner as the controller 80 of the above-described first embodiment, for example. Accordingly, the controller 80 which is a safety circuit can check the operations of the magnetic sensors 40, 40, and 41. As a result, the controller can accurately determine whether or not magnetic particles are contained in the blood 10.

### <Magnetic Particles>

Examples of magnetic particles include particles exhibiting ferromagnetism or paramagnetism. At least a part of the outer circumferential portion of each magnetic particle is modified with a separation-target capturing material which is capable of capturing a specific substance in blood.

The magnetic particles may include: for example, iron, cobalt, nickel, and inorganic compounds thereof; and organically modified inorganic compounds obtained so that these metals or inorganic compounds are modified with organic compounds.

The magnetic particles can capture a specific substance in blood through the separation-target capturing material. A method of capturing a specific component in blood by the separation-target capturing material is not particularly limited. For example, a chemical bonding (such as a molecular biological bonding, an electrical bonding (a van der Waals force, polar attraction, intermolecular forces, and Coulomb force), and the like), adsorption, and trapping using a three-dimensional structure can be appropriately adopted. Magnetic particles may be coated with coating materials including the separation-target capturing material. Also, whole body magnetic particle may be formed from the separation-target capturing material itself. In addition, magnetic particles may be embedded in the separation-target capturing material being formed in a granular shape.

At least a part of the outer circumferential portion of a magnetic particle is preferably coated with a polymer or a silica matrix depending on the component to be separated out.

At least a part of the outer circumferential portion of a magnetic particle may have hydrophobicity or hydrophilicity depending on the component to be separated out.

The average particle diameter of magnetic particles may be greater than or equal to 2 nm. In a case where the average particle diameter of magnetic particles is within the above-described range, the magnetic particles can be efficiently collected by the magnetic field generator included in the magnetic extraction unit 30. In a case where the average particle diameter thereof is greater than or equal to 100 nm, the effect becomes larger, and in a case where the average particle diameter thereof is greater than or equal to 250 nm, the effect becomes even larger.

The average particle diameter of magnetic particles may be less than or equal to 1 mm. In a case where the average particle diameter of magnetic particles is within the above-described range, the magnetic particles have high dispersibility and a large specific surface area. As a result, it is possible to efficiently capture (for example, adsorb) a specific substance in blood. In a case where the average particle diameter thereof is less than or equal to 10 µm, the effect becomes larger, and in a case where the average particle diameter thereof is less than or equal to 5 µm, the effect becomes even larger.

In the present specification, the "average particle diameter" means a particle diameter (also referred to as a median diameter D₅₀) at 50% of cumulative values in a particle size distribution obtained through a laser diffraction/light scattering method.

Animals from which blood to be purified by the blood purification device 1C is derived are not particularly limited. The blood may be derived from humans or non-human animals such as livestock or pets. Examples of non-human animals include dogs, cats, monkeys, pigs, cows, horses, sheep, goats, mice, rats, and birds.

As described above, the blood purification device 1C can extract a specific substance from blood to purify the blood. The blood purification device 1C may be directly connected to an animal to immediately purify blood collected from the animal. Alternatively, the blood purification device 1C can purify blood which has been collected from an animal in advance without being directly connected to the animal. The purified blood may be immediately returned to the individual animal or may be returned to the individual animal after being stored temporarily.

In the present embodiment, examples of specific substances in blood to be removed by magnetic particles include low-molecular-weight compounds, proteins, nucleic acids, and cells. More specific examples thereof include urea, creatinine, uric acid, β2-micro globulin, LDL cholesterol, abnormal antibodies in immune diseases, and substances, such as viruses, bacteria, fungi, and cancer cells, which cause diseases.

### (Fourth Embodiment)

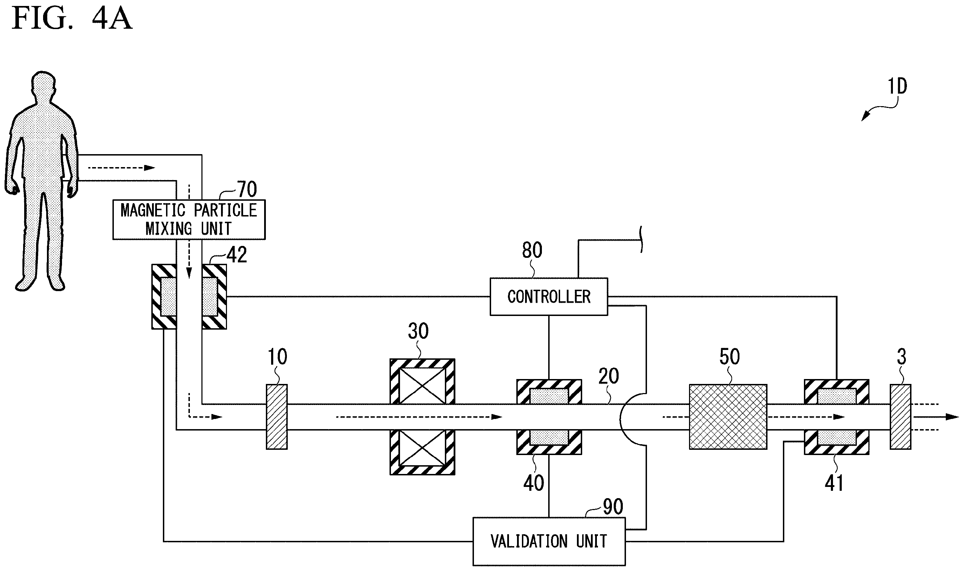

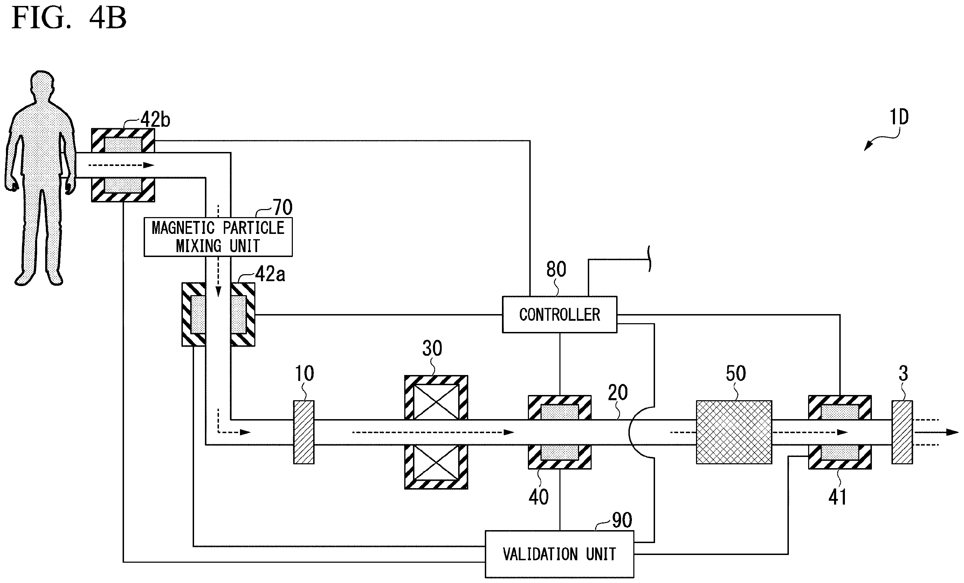

Figs. 4A and 4B are plan views showing configurations of a blood purification device ID according to a fourth embodiment of the present disclosure. In the present embodiment, a case where a flow channel through which blood flows includes no return flow channel and the blood purification device ID includes a controller 80 for processing measurement values of magnetic sensors will be described as an example.

As exemplified in Fig. 4A, the blood purification device ID includes: a main flow channel 20 through which blood 10 flows; a magnetic extraction unit 30 (magnetic extraction means) for collecting magnetic particles contained in the blood 10; and magnetic sensors 40, 41, and 42 for detecting the presence of the magnetic particles in the blood 10; a magnetic particle mixing unit 70 for mixing the magnetic particles into the blood 10; and a controller 80 for processing measurement values of magnetic sensors. In addition, the blood purification device ID includes a filter 50, a connection port (inlet) 2, and a connection port (outlet) 3.

As exemplified in Fig. 4B, the blood purification device ID may include at least one of magnetic sensors 42a and 42b for detecting the presence of magnetic particles in the blood 10. Both the magnetic sensors 42a and 42b are exemplified in Fig. 4B as a specific example, but the blood purification device ID may include only the magnetic sensor 42b.

The magnetic particle mixing unit 70 is located on a preceding portion with respect to the magnetic extraction unit 30, and magnetic particles are mixed into the blood 10 taken out of the body. However, the magnetic particle mixing unit 70 may not be included in a part of the blood purification device ID, and may be implemented outside the blood purification device ID.

In the specific examples shown in Figs. 4A and 4B, the magnetic sensors 42, 42a, and 42b are implemented as parts preceding the magnetic extraction unit 30. The magnetic sensors 42 and 42a may be located on a subsequent portion with respect to the magnetic particle mixing unit 70. The magnetic sensor 42b may be located on a preceding portion with respect to the magnetic particle mixing unit 70.

The magnetic sensor 42 (42a) can measure magnetism indicated by blood after magnetic particles are mixed into the blood. In this case, a magnetism value measured by the magnetic sensor 42 (42a) may be used as a reference value (hereinafter, sometimes referred to as a "first reference value") after charging magnetic particles, for example.

The magnetic sensor 42b can measure magnetism indicated by blood itself before magnetic particles are mixed into the blood. In this case, a magnetism value measured by the magnetic sensor 42b may be used as a reference value (hereinafter, sometimes referred to as a "second reference value") before charging magnetic particles, for example.

The magnetic extraction unit 30, the magnetic sensors 40 and 41 are disposed so as to come into contact with the main flow channel 20. The magnetic sensors 40 and 41 are implemented as parts subsequent to the magnetic extraction unit 30. In one embodiment of the blood purification device ID, the number of magnetic sensors implemented as parts subsequent to the magnetic extraction unit 30 may be two, however the number of magnetic sensor(s) may be one or may be three or more.

The controller 80 is connected to, for example, the magnetic sensors 40, 41, and 42 and an artificial heart-lung machine (not shown in the drawings) (Fig. 4A). The controller 80 may be connected to the magnetic sensors 40, 41, 42a, and 42b and an artificial heart-lung machine (not shown in the drawings) as exemplified in Fig. 4B. Signals, for example, from the magnetic sensors 40, 41, and 42 are input to the controller 80. Signals from the magnetic sensors 40, 41, 42a, and 42b may be input to the controller 80 (Fig. 4B). In addition, as an example, the controller 80 may be configured to be able to output signals to the artificial heart-lung machine. However, the controller 80 may not be directly connected to the magnetic sensors 40, 41, 42, 42a, and 42b and the artificial heart-lung machine. For example, the controller 80 may receive data of measurement values measured by the magnetic sensors 40, 41, 42, 42a, 42b, and the like as input.

The blood 10 containing magnetic particles flows from the connection port 2 into the main flow channel 20, and the magnetic particles are collected by the magnetic extraction unit 30. Subsequently, after the blood 10 has passed through the filter 50, the magnetism of the blood is measured by the magnetic sensor 41.

As one specific example, the controller 80 may execute processing of comparing the magnetism of the blood measured by the magnetic sensor 40 with the magnetism (first reference value) which has been measured by the magnetic sensor 42 (42a) and is indicated by the blood containing magnetic particles. In this case, the controller 80 can, for example, output the results of the processing to an appropriate display device or the like. In addition, the controller 80 may output signals for controlling an operation of the artificial heart-lung machine according to results obtained by comparing a specific reference value (or a range of reference values) with a difference between the magnetism of the blood measured by the magnetic sensor 41 and the magnetism which has been measured by the magnetic sensor 42 (42a) and is indicated by the blood containing magnetic particles.

The controller 80 may control whether or not to allow the blood 10 to flow into the artificial heart-lung machine according to the results obtained by comparing the magnetism of the blood measured by the magnetic sensor 41 with the first reference value measured by the magnetic sensor 42 (42a), for example.

The controller 80 may allow the blood 10 to flow into the artificial heart-lung machine in a case where, for example, the magnetism of the blood measured by the magnetic sensor 41 is reduced to a certain specific reference value or more (that is, in a case where the magnetic particles are captured by the magnetic extraction unit 30 and the amount of magnetic particles in the blood is sufficiently reduced) compared to the first reference value.

On the other hand, in a case where the controller 80 determines that the amount of magnetic particles in the blood is not sufficiently reduced, the controller may control the blood 10 not to flow into the artificial heart-lung machine. Specifically, in a case where, for example, the magnetism of the blood measured by the magnetic sensor 41 is larger than the first reference value, the controller 80 may control the artificial heart-lung machine or the like so as to stop the blood 10 from flowing into the artificial heart-lung machine. In addition, in a case where, for example, the difference between the first reference value and the magnetism of the blood measured by the magnetic sensor 41 is not included in a range of specific reference values (for example, in a case where the difference therebetween is smaller than a certain reference value), the controller 80 may control the artificial heart-lung machine or the like so as to stop the blood 10 from flowing into the artificial heart-lung machine. Accordingly, the controller 80 can prevent the blood 10, which contains magnetic particles at an amount out of a range of certain specific reference values, from flowing into the artificial heart-lung machine, for example. The reference value may be appropriately determined from the viewpoint of, for example, safety to the human body (the same applies to the embodiments to be described below).

As another specific example, the controller 80 may execute processing of comparing the magnetism of the blood measured by the magnetic sensor 40 with the magnetism (second reference value) which has been measured by the magnetic sensor 42b and is indicated by the blood itself containing no magnetic particles. In this case, the controller 80 can, for example, output the results of the processing to an appropriate display device or the like.

The controller 80 may control whether or not to allow the blood 10 to flow into the artificial heart-lung machine according to the results obtained by comparing the magnetism of the blood measured by the magnetic sensor 41 with the second reference value measured by the magnetic sensor 42b, for example.

The controller 80 may allow the blood 10 to flow into the artificial heart-lung machine in a case where, for example, the magnetism of the blood measured by the magnetic sensor 41 is less than or equal to the second reference value (in a case where the amount of magnetic particles in the blood is sufficiently reduced).

On the other hand, in a case where, for example, the magnetism of the blood measured by the magnetic sensor 41 is higher than the second reference value, the controller 80 may control the artificial heart-lung machine so as to stop the blood 10 from flowing into the artificial heart-lung machine. In a case where, for example, the difference between the second reference value and the magnetism of the blood measured by the magnetic sensor 41 is not included in a range of a specific reference value (for example, in a case where the difference therebetween is larger than a certain reference value), the controller 80 may control the artificial heart-lung machine so as to stop the blood 10 from flowing into the artificial heart-lung machine. Accordingly, the controller 80 can prevent the blood 10, which contains magnetic particles at an amount out of a range of certain specific reference values, from flowing into the artificial heart-lung machine, for example.

As described above, the controller 80 can control whether or not to allow the blood 10 to flow into the artificial heart-lung machine according to the results obtained by comparing the magnetism of the blood measured by the magnetic sensor 41 with the first reference value measured by the magnetic sensor 42 (magnetic sensor 42a) or the second reference value measured by the magnetic sensor 42b. As an example, the controller 80 may control whether or not to allow the blood 10 to flow into the artificial heart-lung machine depending on whether or not a difference between the magnetism of the blood measured by the magnetic sensor 41 and the first reference value or a difference between the magnetism of the blood measured by the magnetic sensor 41 and the second reference value is included in a specific range as described above.

In this blood purification device ID, it is possible to more accurately measure the magnetism of the blood 10 by providing the controller 80 and to confirm that magnetic particles have been sufficiently separated and removed from blood before purified blood is returned to the body.

The filter 50 can capture, for example, the magnetic particles, which could not be removed by the magnetic extraction unit 30, and foreign substances contained in the blood 10.

### <Magnetic Extraction Unit>

The magnetic extraction unit 30 can collect magnetic particles in blood flowing through the main flow channel 20 with a magnetic force. Accordingly, a specific substance captured by the magnetic particles can be extracted from blood.

Known methods can be employed to implement the magnetic extraction unit 30. For example, magnetic separation method disclosed in Patent Literature 1 can be used.

The collection of magnetic particles from blood may be performed, for example, by taking out the magnetic particles into a flow channel connected to the main flow channel 20, or magnetic particles may be collected in a collection unit located on an inner wall of the main flow channel 20.

The magnetic extraction unit 30 may have magnetic field generator (not shown in the drawings) for generating a magnetic field. Magnetic particles are collected from the blood 10 flowing through the main flow channel 20 due to this magnetic field. The magnetic field generator may be, for example, a permanent magnet or a variable magnetic flux magnet, or may be, for example, an induced magnetic field.

The magnetic extraction unit 30 may have a member, such as a magnetic shield layer, capable of shielding a magnetic field. Accordingly, it is possible to suppress or prevent a leakage magnetic field from the magnetic extraction unit 30.

The magnetic field generator may be disposed outside the main flow channel 20 and free of contact with the blood 10. Since the magnetic field generator does not come into contact with the blood 10, coagulation of the blood 10 and contamination with foreign substances can be suppressed.

### <Magnetic Sensor>

The magnetic sensors 40, 41, 42 (42a), and 42b can detect the presence of magnetic particles by detecting a leakage magnetic field generated from magnetic particles in blood flowing through the main flow channel 20.

The magnetic sensors 40, 41, 42 (42a), and 42b may be disposed outside the main flow channel 20 and free of contact with the blood 10. Since the magnetic sensors 40, 41, and 42 are free of contact with the blood 10, coagulation of the blood 10 and contamination with foreign substances can be suppressed.

Known magnetic sensors can be used as the magnetic sensors 40, 41, 42 (42a), and 42b. For example, coil-type sensors may be used, Hall sensors may be used, or elements using magnetic resistance may be used. It is possible to determine whether or not the blood 10 flowing through the main flow channel 20 contains magnetic particles based on measurement values of magnetism indicated by the magnetic sensors 40, 41, 42 (42a), and 42b. The magnetic sensors 40, 41, 42 (42a), and 42b may be the same or different types of magnetic sensors.

A coil-type sensor measures and detects a magnetic field of magnetic particles which are passing through using an induction coil when a magnetic particle-containing sample is passed through the induction coil. The coil-type sensor has an advantage of being able to be easily installed.

A Hall sensor detects a magnetic field using the Hall effect and has advantages of being inexpensive and able to be easily installed.

A magnetoresistive element is an element utilizing a phenomenon in which electrical resistance changes due to an effect of a magnetic field, and includes an anisotropic magnetoresistive effect (AMR) element, a giant magnetoresistive (GMR) element, a tunnel magnetoresistive resistance (TMR) element, and the like. A GMR element or a TMR element has advantages that the temperature change or the change with time is small and the sensitivity (MR ratio) is large.

The magnetic sensors 40, 41, 42 (42a), and 42b may have a member, such as a magnetic shield layer, capable of shielding a magnetic field. Accordingly, it is possible to prevent failures such as false detection due to influence of a magnetic field generated from the magnetic extraction unit 30.

### <Blood Pump>

The blood purification device ID may include a blood pump for taking out blood from the body. Alternatively, an externally provided blood pump may be used separately from the blood purification device of the present disclosure.

In this blood purification device ID, for example, the blood 10 containing magnetic particles modified with the separation-target capturing material, which is capable of capturing a specific substance in the blood, is allowed to flow through the main flow channel 20 through which the blood 10 can flow. In addition, the magnetic extraction unit 30 capable of generating a magnetic field collects at least the portion of the magnetic particles contained in the blood 10. The magnetic sensors 40 and 41 capable of detecting the presence of the magnetic particles detect whether or not the magnetic particles have remained in the blood after at least the portion of the magnetic particles are collected by the magnetic extraction unit 30.

Although the fourth embodiment of the present disclosure has been described in detail above, the technology according to the present disclosure is not limited to the fourth embodiment and various modifications and changes can be made within the scope of the gist of the present disclosure disclosed in the claims.

For example, the temperature of the entire blood purification device ID in the fourth embodiment may be kept constant or within a specific temperature range. For example, an exterior cover which externally covers the entire blood purification device and is composed of a heat insulating material or the like may be provided in the blood purification device. The measurement values of the magnetic sensors are stabilized by keeping the temperature of the entire device constant. In addition, the temperature of the entire blood purification device is preferably kept the same as body temperature. For example, the blood purification device may further include: a temperature sensor (not shown in the drawings) that detects the internal temperature of the above-described exterior cover; and a heating unit (not shown in the drawings) of a heater or the like that heats the interior of the above-described exterior cover according to signals from the controller based on the temperature sensor. Accordingly, it is possible to stabilize the measurement values of the magnetic sensors and to maintain the quality of blood flowing through the blood purification device for a long period of time.

In addition, as shown in Figs. 4A and 4B, the blood purification device ID may further include a validation unit 90 which determines whether or not the magnetic sensors 40, 41, 42 (42a), and 42b are operating correctly. For example, the validation unit 90 may be connected to the magnetic sensors 40, 41, 42 (42a), and 42b and determine whether the magnetic sensors 40, 41, 42 (42a), and 42b are operating correctly on a basis of the outputs (magnetic values of blood) from the magnetic sensors. The validation unit may be configured in the same manner as the validation unit 90 of the above-described first embodiment, for example.

In addition, the blood purification device ID may further include a controller 80 which is connected to the magnetic sensors 40, 41, 42 (42a), and 42b and determines whether or not magnetic particles are contained in the blood 10 on a basis of the outputs from the magnetic sensors. The controller 80 may check operation states of the magnetic sensors 40, 41, 42 (42a), and 42b which have been determined by the validation unit 90. The controller 80 of this third embodiment can be configured in the same manner as the controller 80 of the above-described first embodiment, for example. Accordingly, the controller 80 which is a safety circuit can check the operations of the magnetic sensors 40, 41, 42 (42a), and 42b. As a result, the controller can accurately determine whether or not magnetic particles are contained in the blood 10.

### <Magnetic Particles>

Examples of magnetic particles include particles exhibiting ferromagnetism or paramagnetism. At least a part of the outer circumferential portion of each magnetic particle is modified with a separation-target capturing material which is capable of capturing a specific substance in blood.

The magnetic particles may include: for example, iron, cobalt, nickel, and inorganic compounds thereof; and organically modified inorganic compounds obtained so that these metals or inorganic compounds are modified with organic compounds.

The magnetic particles can capture a specific substance in blood through the separation-target capturing material. A method of capturing a specific component in blood by the separation-target capturing material is not particularly limited. For example, a chemical bonding (such as a molecular biological bonding, an electrical bonding (a van der Waals force, polar attraction, intermolecular forces, and Coulomb force, and the like)), adsorption, and trapping using a three-dimensional structure can be appropriately adopted. Magnetic particles may be coated with coating materials including the separation-target capturing material. Also, whole body magnetic particle may be formed from the separation-target capturing material itself.. In addition, magnetic particles may be embedded in the separation-target capturing material being formed in a granular shape.

At least a part of the outer circumferential portion of a magnetic particle is preferably coated with a polymer or a silica matrix depending on the component to be separated out.

At least a part of the outer circumferential portion of a magnetic particle may have hydrophobicity or hydrophilicity depending on the component to be separated out.

The average particle diameter of magnetic particles may be greater than or equal to 2 nm. In a case where the average particle diameter of magnetic particles is within the above-described range, the magnetic particles can be efficiently collected by the magnetic field generator included in the magnetic extraction unit 30. In a case where the average particle diameter thereof is greater than or equal to 100 nm, the effect becomes larger, and in a case where the average particle diameter thereof is greater than or equal to 250 nm, the effect becomes even larger.

The average particle diameter of magnetic particles may be less than or equal to 1 mm. In a case where the average particle diameter of magnetic particles is within the above-described range, the magnetic particles have high dispersibility and a large specific surface area. As a result, it is possible to efficiently capture (for example, adsorb) a specific substance in blood. In a case where the average particle diameter thereof is less than or equal to 10 µm, the effect becomes larger, and in a case where the average particle diameter thereof is less than or equal to 5 µm, the effect becomes even larger.

In the present specification, the "average particle diameter" means a particle diameter (also referred to as a median diameter D₅₀) at 50% of cumulative values in a particle size distribution obtained through a laser diffraction/light scattering method.

Animals from which blood to be purified by the blood purification device ID is derived are not particularly limited. The blood may be derived from humans or non-human animals such as livestock or pets. Examples of non-human animals include dogs, cats, monkeys, pigs, cows, horses, sheep, goats, mice, rats, and birds.

As described above, the blood purification device ID can extract a specific substance from blood to purify the blood. The blood purification device ID may be directly connected to an animal to immediately purify blood collected from the animal. Alternatively, the blood purification device ID can purify blood which has been collected from an animal in advance without being directly connected to the animal. The purified blood may be immediately returned to the individual animal or may be returned to the individual animal after being stored temporarily.

In the present embodiment, examples of specific substances in blood to be removed by magnetic particles include low-molecular-weight compounds, proteins, nucleic acids, and cells. More specific examples thereof include urea, creatinine, uric acid, β2-micro globulin, LDL cholesterol, abnormal antibodies in immune diseases, and substances, such as viruses, bacteria, fungi, and cancer cells, which cause diseases.

### (Fifth Embodiment)

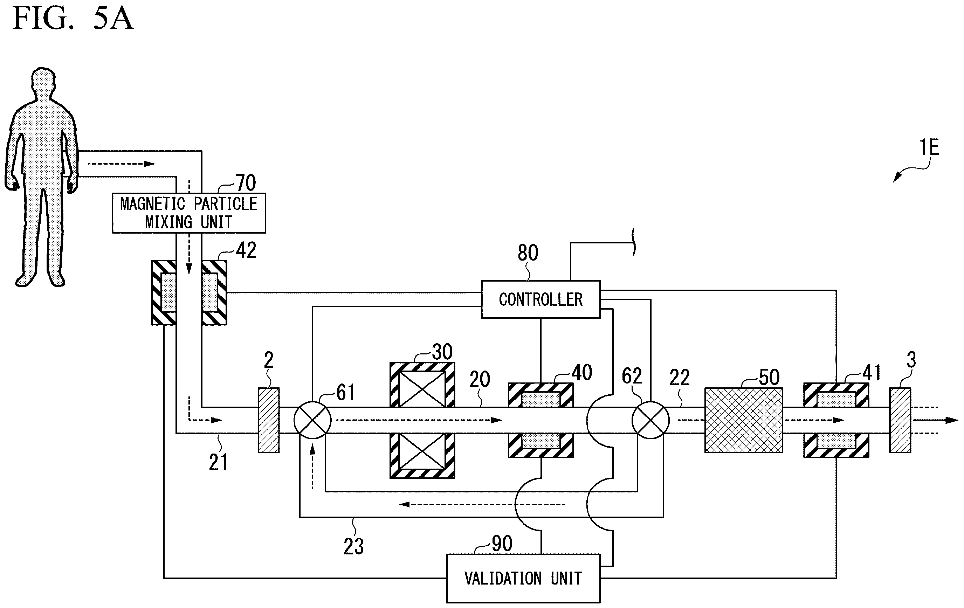

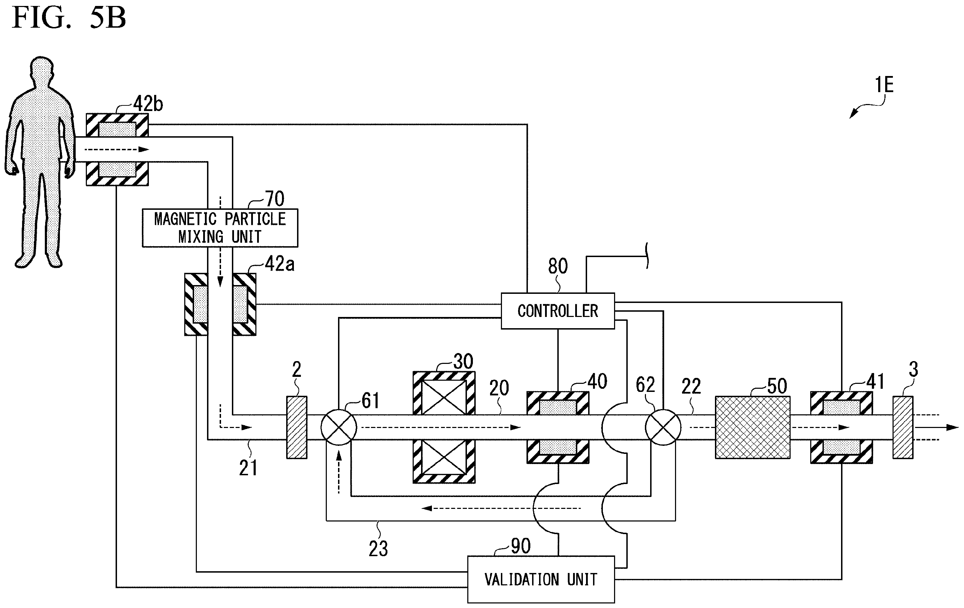

Figs. 5A and 5B are plan views showing configurations of a blood purification device IE according to a fifth embodiment of the present disclosure. In the present embodiment, a case where the blood purification device IE includes a flow channel through which blood flows and a return flow channel and a controller 80 for operating a flow channel selection unit will be described as an example.

As exemplified in Fig. 5A, the blood purification device IE includes: main flow channels 20, 21, and 22 through which blood 10 flows; a magnetic extraction unit 30 (magnetic extraction means) for collecting magnetic particles contained in the blood 10; and magnetic sensors 40, 41, and 42 for detecting the presence of the magnetic particles in the blood 10; a magnetic particle mixing unit 70 for mixing the magnetic particles into the blood 10; and a controller 80 for processing measurement values of magnetic sensors. In addition, the blood purification device IE includes: a return flow channel 23 for recirculating the blood 10; a first flow channel selection unit 61 (first flow channel selection means) for selecting a flow channel; and a second flow channel selection unit 62 (second flow channel selection means) for selecting a flow channel. Furthermore, the blood purification device IE includes a filter 50, a connection port (inlet) 2, and a connection port 3.

As exemplified in Fig. 5B, the blood purification device IE may include at least one of magnetic sensors 42a and 42b for detecting the presence of magnetic particles in the blood 10. Both the magnetic sensors 42a and 42b are exemplified in Fig. 5B as a specific example, but the blood purification device IE may include only the magnetic sensor 42b.

The magnetic particle mixing unit 70 is located on a preceding portion with respect to the magnetic extraction unit 30, and magnetic particles are mixed into the blood 10. However, the magnetic particle mixing unit 70 may be implemented in the blood purification device IE, or may be implemented outside the blood purification device IE.

In the specific examples shown in Figs. 5A and 5B, the magnetic sensors 42, 42a, and 42b are implemented as parts preceding the magnetic extraction unit 30. The magnetic sensors 42 and 42a may be located on a subsequent portion with respect to the magnetic particle mixing unit 70. The magnetic sensor 42b may be located on a preceding portion with respect to the magnetic particle mixing unit 70.

The magnetic sensor 42 (42a) can measure magnetism indicated by blood after magnetic particles are mixed into the blood. In this case, a magnetism value measured by the magnetic sensor 42 (42a) may be used as a reference value (a "first reference value") after charging magnetic particles, for example.

The magnetic sensor 42b can measure magnetism indicated by blood itself containing no magnetic particles before magnetic particles are mixed into the blood. In this case, a magnetism value measured by the magnetic sensor 42b may be used as a reference value (a "first reference value") before charging magnetic particles, for example.

The first flow channel selection unit 61 and the second flow channel selection unit 62 are, for example, three-way solenoid valves. Not limited to the above, the first flow channel selection unit 61 and the second flow channel selection unit 62 may be realized with an appropriate configuration (for example, an actuator or a valve) capable of selecting a flow channel of blood using control signals output from the controller 80, for example.

The magnetic extraction unit 30, the magnetic sensors 40 and 41 are disposed so as to come into contact with the main flow channel 20. The magnetic sensors 40 and 41 are implemented as parts subsequent to the magnetic extraction unit 30. In one embodiment of the blood purification device 1E, the number of magnetic sensors implemented as parts subsequent to the magnetic extraction unit 30 may be two, however, the number of magnetic sensor(s) may be one or may be three or more.

The controller 80 is connected to the magnetic sensors 40, 41, and 42, the first flow channel selection unit 61, the second flow channel selection unit 62, and an artificial heart-lung machine (not shown in the drawings) (Fig. 5A). The controller 80 may be connected to the magnetic sensors 40, 41, 42a, and 42b, the first flow channel selection unit 61, the second flow channel selection unit 62, and an artificial heart-lung machine (not shown in the drawings) as exemplified in Fig. 5B. Signals, for example, from the magnetic sensors 40, 41, and 42 are input to the controller 80. Signals from the magnetic sensors 40, 41, 42a, and 42b may be input to the controller 80 (Fig. 5B). In addition, as an example, the controller 80 may output signals to the first flow channel selection unit 61, the second flow channel selection unit 62, and the artificial heart-lung machine. However, the controller 80 may not be directly connected to the magnetic sensors 40, 41, 42, 42a, and 42b and the artificial heart-lung machine. For example, the controller 80 may receive data of measurement values measured by the magnetic sensors 40, 41, 42, 42a, 42b, and the like as input.

First, the controller 80 outputs signals to the first flow channel selection unit 61 and operates the first flow channel selection unit 61 to allow communication between the main flow channel 21 and the main flow channel 20. In addition, the controller 80 outputs signals to the second flow channel selection unit 62 and operates the second flow channel selection unit 62 to allow communication between the main flow channel 20 and the return flow channel 23 and block communication between the main flow channels 20 and 22. Accordingly, the blood 10 containing magnetic particles flows from the connection port 2 into the main flow channel 21.

After the blood 10 containing magnetic particles flows in from the connection port 2, the controller 80 operates the first flow channel selection unit 61 to allow communication between the main flow channel 20 and the return flow channel 23. At this time, the second flow channel selection unit 62 allows a state where the main flow channel 20 is allowed to communicate with the return flow channel 23 to continue.

The blood 10 circulates in the main flow channel 20 and the return flow channel 23. During that period, the magnetic particles are collected by the magnetic extraction unit 30, and the magnetism generated from the blood 10 is measured by the magnetic sensor 40.

As one specific example, the controller 80 may execute processing of comparing the magnetism of the blood measured by the magnetic sensor 40 with the magnetism (first reference value) which has been measured by the magnetic sensor 42 (42a) and is indicated by the blood containing magnetic particles. In this case, the controller 80 can, for example, output the results of the processing to an appropriate display device or the like. In addition, the controller 80 may control at least one of the first flow channel selection unit 61 and the second flow channel selection unit 62 according to the results obtained by comparing the magnetism of the blood measured by the magnetic sensor 40 with the first reference value measured by the magnetic sensor 42 (42a).

In a case where, for example, the magnetism of the blood measured by the magnetic sensor 40 is reduced to a certain specific reference value or more (that is, in a case where the magnetic particles are captured by the magnetic extraction unit 30 and the amount of magnetic particles in the blood is sufficiently reduced) compared to the first reference value, the controller 80 may control at least one of the first flow channel selection unit 61 and the second flow channel selection unit 62 so as to allow communication between the main flow channel 20 and the main flow channel 22. Specifically, for example, the controller 80 outputs signals to the second flow channel selection unit 62 and operates the second flow channel selection unit 62 to allow communication between the main flow channel 20 and the main flow channel 22. At this time, the controller 80 allows the state of the first flow channel selection unit 61 allowing communication between the main flow channel 20 and the return flow channel 23 to continue. Then, the blood 10 flows into the main flow channel 22 and passes through the filter 50.

On the other hand, in a case where the controller 80 determines that the amount of magnetic particles in the blood is not sufficiently reduced, the controller may control at least one of the first flow channel selection unit 61 and the second flow channel selection unit 62 so as to maintain the state where the main flow channel 20 and the return flow channel 23 communicate with each other. Specifically, in a case where, for example, the controller 80 determines that the magnetism of the blood measured by the magnetic sensor 40 is larger than the first reference value measured by the magnetic sensor 42 (42a), the controller may control at least one of the first flow channel selection unit 61 and the second flow channel selection unit 62 to allow the state where the main flow channel 20 is allowed to communicate with the return flow channel 23 to continue. In addition, in a case where, for example, the difference between the first reference value and the magnetism of the blood measured by the magnetic sensor 40 is not included in a range of specific reference values (for example, in a case where the difference therebetween is smaller than a certain standard, the controller 80 may control at least one of the first flow channel selection unit 61 and the second flow channel selection unit 62 so as to maintain the state where the main flow channel 20 and the return flow channel 23 communicate with each other. The first flow channel selection unit 61 and the second flow channel selection unit 62 allow the state where the main flow channel 20 is allowed to communicate with the return flow channel 23 to continue according to the control signals from the controller 80. Accordingly, the controller 80 can prevent the blood 10, which contains magnetic particles at an amount out of a range of certain specific reference values, from flowing into the main flow channel 22, for example.

As another specific example, the controller 80 may execute processing of comparing the magnetism of the blood measured by the magnetic sensor 40 with the magnetism (second reference value) which has been measured by the magnetic sensor 42b and is indicated by the blood itself containing no magnetic particles. The controller 80 may control, for example, at least one of the first flow channel selection unit 61 and the second flow channel selection unit 62 according to the results obtained by comparing the magnetism of the blood measured by the magnetic sensor 40 with the second reference value measured by the magnetic sensor 42b.

Specifically, the controller 80 may execute the following processing in a case where, for example, the magnetism of the blood measured by the magnetic sensor 40 is less than or equal to the second reference value (in a case where the amount of magnetic particles in the blood is sufficiently reduced). That is, the controller 80 may control at least one of the first flow channel selection unit 61 and the second flow channel selection unit 62 so as to allow communication between the main flow channel 20 and the main flow channel 22. For example, the controller 80 outputs signals to the second flow channel selection unit 62 and operates the second flow channel selection unit 62 to allow communication between the main flow channel 20 and the main flow channel 22. At this time, the controller 80 allows the state of the first flow channel selection unit 61 allowing communication between the main flow channel 20 and the return flow channel 23 to continue. Then, the blood 10 flows into the main flow channel 22 and passes through the filter 50.

On the other hand, in a case where, for example, the magnetism of the blood measured by the magnetic sensor 40 is larger than the second reference value (that is, in a case where the amount of magnetic particles in the blood is not sufficiently reduced), the controller 80 may control at least one of the first flow channel selection unit 61 and the second flow channel selection unit 62 so as to maintain the state where the main flow channel 20 and the return flow channel 23 communicate with each other. In a case where, for example, the difference between the second reference value and the magnetism of the blood measured by the magnetic sensor 40 is not included in a range of specific reference values (for example, in a case where the difference therebetween is larger than a certain reference value), the controller 80 may control at least one of the first flow channel selection unit 61 and the second flow channel selection unit 62 so as to maintain the state where the main flow channel 20 and the return flow channel 23 communicate with each other. The first flow channel selection unit 61 and the second flow channel selection unit 62 allow the state where the main flow channel 20 is allowed to communicate with the return flow channel 23 to continue according to the control signals from the controller 80. Accordingly, the controller 80 can prevent the blood 10, which contains magnetic particles at an amount out of a range of certain specific reference values, from flowing into the main flow channel 22, for example.

As described above, the controller 80 can control at least one of the first flow channel selection unit 61 and the second flow channel selection unit 62 according to the results obtained by comparing the magnetism of the blood measured by the magnetic sensor 40 with the first reference value measured by the magnetic sensor 42 (magnetic sensor 42a). In addition, the controller 80 can control at least one of the first flow channel selection unit 61 and the second flow channel selection unit 62 according to the results obtained by comparing the magnetism of the blood measured by the magnetic sensor 40 with the second reference value measured by the magnetic sensor 42b. As an example, the controller 80 may control at least one of the first flow channel selection unit 61 and the second flow channel selection unit 62 depending on whether or not a difference between the magnetism of the blood measured by the magnetic sensor 40 and the first reference value or a difference between the magnetism of the blood measured by the magnetic sensor 40 and the second reference value is included in a specific range as described above. Accordingly, the controller 80 can control the blood flow according to the measurement results of the magnetic sensors.

In addition, the controller 80 can determine whether or not the blood 10 flowing through the main flow channel 22, that is, the blood 10 after passing through the filter 50 contains magnetic particles based on the magnetism of the blood measured by the magnetic sensor 41 and the magnetism (first reference value) which has been measured by the magnetic sensor 42 (42a) and is indicated by blood containing magnetic particles. As an example, in a case where the magnetism of the blood measured by the magnetic sensor 41 is reduced to a certain specific reference value or more compared to the first reference value, the controller 80 may determine that the amount of magnetic particles contained in the blood 10 after passing through the filter 50 is sufficiently reduced. In addition, in a case where, for example, the difference between the magnetism of the blood measured by the magnetic sensor 41 and the first reference value is included in a certain specific range (for example, in a case where the difference therebetween is larger than a certain reference value), the controller 80 may determine that the amount of magnetic particles contained in the blood 10 after passing through the filter 50 is sufficiently reduced.

Similarly, the controller 80 can determine whether or not the blood 10 flowing through the main flow channel 22, that is, the blood 10 after passing through the filter 50 contains magnetic particles based on the magnetism of the blood measured by the magnetic sensor 41 and the magnetism (second reference value) which has been measured by the magnetic sensor 42b and is indicated by blood containing no magnetic particles. As an example, in a case where the magnetism of the blood measured by the magnetic sensor 41 is less than or equal to the second reference value, the controller 80 may determine that the amount of magnetic particles contained in the blood 10 after passing through the filter 50 is sufficiently reduced. In addition, in a case where, for example, the difference between the magnetism of the blood measured by the magnetic sensor 41 and the second reference value is included in a certain specific range (for example, in a case where the difference therebetween is greater than or equal to a certain reference value), the controller 80 may determine that the amount of magnetic particles contained in the blood 10 after passing through the filter 50 is sufficiently reduced.

In a case where the controller 80 determines that the blood 10 flowing through the main flow channel 22 contains no magnetic particles (or that the amount of magnetic particles is sufficiently reduced), the state where the main flow channel 20 and the main flow channel 22 communicate with each other is continued. In this case, the blood 10 flows out of the connection port 3. On the other hand, it is determined that the blood 10 flowing through the main flow channel 22 contains magnetic particles (or that the amount of magnetic particles is not sufficiently reduced), the controller 80 may output signals for controlling the operation the artificial heart-lung machine. For example, the controller 80 may control the artificial heart-lung machine so as to stop the blood 10 from flowing into the artificial heart-lung machine. At this time, the controller 80 may control at least one of the first flow channel selection unit 61 and the second flow channel selection unit 62 to block communication between the main flow channel 22 and the return flow channel 23 and to switch the flow channels into a state where the main flow channel 20 is allowed to communicate with the return flow channel 23. Accordingly, the controller 80 can prevent the blood 10, which contains magnetic particles, from flowing into the artificial heart-lung machine.

In this blood purification device IE, the controller 80 operates the first flow channel selection unit 61 and the second flow channel selection unit 62 based on the measurement values of the magnetic sensors 40, 42 (42a), and 42b. Therefore, magnetic particles can be sufficiently separated and removed from blood before purified blood is returned to the body.

The filter 50 can capture, for example, the magnetic particles, which could not be removed by the magnetic extraction unit 30, and foreign substances contained in the blood 10.

### <Magnetic Extraction Unit>

The magnetic extraction unit 30 can collect magnetic particles in blood flowing through the main flow channel 20 with a magnetic force. Accordingly, a specific substance captured by the magnetic particles can be extracted from blood.

Known methods can be employed to implement the magnetic extraction unit 30. For example, magnetic separation method disclosed in Patent Literature 1 can be used.

The collection of magnetic particles from blood may be performed, for example, by taking out the magnetic particles into a flow channel connected to the main flow channel 20, or magnetic particles may be collected in a collection unit located on an inner wall of the main flow channel 20.

The magnetic extraction unit 30 may have magnetic field generator for generating a magnetic field. Magnetic particles are collected from the blood 10 flowing through the main flow channel 20 due to this magnetic field. The magnetic field generator may be, for example, a permanent magnet or a variable magnetic flux magnet, or may be, for example, an induced magnetic field.

The magnetic extraction unit 30 may have a member, such as a magnetic shield layer, capable of shielding a magnetic field. Accordingly, it is possible to suppress or prevent a leakage magnetic field from the magnetic extraction unit 30.

The magnetic field generator may be disposed outside the main flow channel 20 and free of contact with the blood 10. Since the magnetic field generator does not come into contact with the blood 10, coagulation of the blood 10 and contamination with foreign substances can be suppressed.

### <Magnetic Sensor>

The magnetic sensors 40, 41, 42 (42a), and 42b can detect the presence of magnetic particles by detecting a leakage magnetic field generated from magnetic particles in blood flowing through the main flow channel 20.

The magnetic sensors 40, 41, 42 (42a), and 42b may be disposed outside the main flow channel 20 and free of contact with the blood 10. Since the magnetic sensors 40, 41, 42 (42a), and 42b are free of contact with the blood 10, coagulation of the blood 10 and contamination with foreign substances can be suppressed.

Known magnetic sensors can be used as the magnetic sensors 40, 41, 42 (42a), and 42b. For example, coil-type sensors may be used, Hall sensors may be used, or elements using magnetic resistance may be used. It is possible to determine whether or not the blood 10 flowing through the main flow channel 20 contains magnetic particles based on measurement values of magnetism indicated by the magnetic sensors 40, 41, 42 (42a), and 42b. The magnetic sensors 40, 41, 42 (42a), and 42b may be the same or different types of magnetic sensors.

A coil-type sensor measures and detects a magnetic field of magnetic particles which are passing through using an induction coil when a magnetic particle-containing sample is passed through the induction coil. The coil-type sensor has an advantage of being able to be easily installed.

A Hall sensor detects a magnetic field using the Hall effect and has advantages of being inexpensive and able to be easily installed.

A magnetoresistive element is an element utilizing a phenomenon in which electrical resistance changes due to an effect of a magnetic field, and includes an anisotropic magnetoresistive effect (AMR) element, a giant magnetoresistive (GMR) element, a tunnel magnetoresistive resistance (TMR) element, and the like. A GMR element or a TMR element has advantages that the temperature change or the change with time is small and the sensitivity (MR ratio) is large.

The magnetic sensors 40, 41, 42 (42a), and 42b may have a member, such as a magnetic shield layer, capable of shielding a magnetic field. Accordingly, it is possible to prevent failures such as false detection due to influence of a magnetic field generated from the magnetic extraction unit 30.

### <Blood Pump>

The blood purification device IE may be connected to a blood pump capable of making blood flow. The blood pump may be, for example, used for taking out blood from the body or used for circulating blood taken out of the body. Alternatively, an externally provided blood pump may be used separately from the blood purification device of the present disclosure.

In this blood purification device 1E, for example, the blood 10 containing magnetic particles modified with the separation-target capturing material, which is capable of capturing a specific substance in the blood, is allowed to flow through the main flow channels 20, 21, and 22 through which the blood 10 can flow. In addition, the magnetic extraction unit 30 capable of generating a magnetic field collects at least the portion of the magnetic particles contained in the blood 10. The magnetic sensors 40 and 41 capable of detecting the presence of the magnetic particles detect whether or not the magnetic particles have remained in the blood after at least the portion of the magnetic particles are collected by the magnetic extraction unit 30.

Although the fifth embodiment of the present disclosure has been described in detail above, the technology according to the present disclosure is not limited to the fifth embodiment and various modifications and changes can be made within the scope of the gist of the present disclosure disclosed in the claims.

For example, the temperature of the entire blood purification device IE in the fifth embodiment may be kept constant or within a specific temperature range. For example, an exterior cover which externally covers the entire blood purification device and is composed of a heat insulating material or the like may be provided in the blood purification device. The measurement values of the magnetic sensors are stabilized by keeping the temperature of the entire device constant. In addition, the temperature of the entire blood purification device is preferably kept the same as body temperature. For example, the blood purification device may further include: a temperature sensor (not shown in the drawings) that detects the internal temperature of the above-described exterior cover; and a heating unit (not shown in the drawings) of a heater or the like that heats the interior of the above-described exterior cover according to signals from the controller based on the temperature sensor. Accordingly, it is possible to stabilize the measurement values of the magnetic sensors and to maintain the quality of blood flowing through the blood purification device for a long period of time.

In addition, as exemplified in Figs. 5A and 5B, the blood purification device IE may further include a validation unit 90 which determines whether or not the magnetic sensors 40, 41, 42 (42a), and 42b are operating correctly. For example, the validation unit 90 may be connected to the magnetic sensors 40, 41, 42 (42a), and 42b and determine whether the magnetic sensors 40, 41, 42 (42a), and 42b are operating correctly on a basis of the outputs (magnetic values of blood) from the magnetic sensors. The validation unit may be configured in the same manner as the validation unit 90 of the above-described first embodiment, for example.

In addition, the blood purification device IE may further include a controller which is connected to the magnetic sensors 40, 41, 42 (42a), and 42b and determines whether or not magnetic particles are contained in the blood 10 on a basis of the outputs from the magnetic sensors. The controller 80 may check operation states of the magnetic sensors 40, 41, 42 (42a), and 42b which have been determined by the validation unit 90. Accordingly, the controller 80 which is a safety circuit can check the operations of the magnetic sensors 40, 41, 42 (42a), and 42b. As a result, the controller can accurately determine whether or not magnetic particles are contained in the blood 10.

### <Magnetic Particles>

Examples of magnetic particles include particles exhibiting ferromagnetism or paramagnetism. At least a part of the outer circumferential portion of each magnetic particle is modified with a the separation-target capturing material which is capable of capturing a specific substance in blood.

The magnetic particles may include: for example, iron, cobalt, nickel, and inorganic compounds thereof; and organically modified inorganic compounds obtained so that these metals or inorganic compounds are modified with organic compounds.

The magnetic particles can capture a specific substance in blood through the separation-target capturing material. A method of capturing a specific component in blood by the separation-target capturing material is not particularly limited. For example, a chemical bonding (such as a molecular biological bonding, an electrical bonding (a van der Waals force, polar attraction, intermolecular forces, and Coulomb force, and the like)), adsorption, and trapping using a three-dimensional structure can be appropriately adopted. Magnetic particles may be coated with coating materials including the separation-target capturing material. Also, whole body magnetic particle may be formed from the separation-target capturing material itself. In addition, magnetic particles may be embedded in the separation-target capturing material being formed in a granular shape.

At least a part of the outer circumferential portion of a magnetic particle is preferably coated with a polymer or a silica matrix depending on the component to be separated out.

At least a part of the outer circumferential portion of a magnetic particle may have hydrophobicity or hydrophilicity depending on the component to be separated out.

The average particle diameter of magnetic particles may be greater than or equal to 2 nm. In a case where the average particle diameter of magnetic particles is within the above-described range, the magnetic particles can be efficiently collected by the magnetic field generator included in the magnetic extraction unit 30. In a case where the average particle diameter thereof is greater than or equal to 100 nm, the effect becomes larger, and in a case where the average particle diameter thereof is greater than or equal to 250 nm, the effect becomes even larger.

The average particle diameter of magnetic particles may be less than or equal to 1 mm. In a case where the average particle diameter of magnetic particles is within the above-described range, the magnetic particles have high dispersibility and a large specific surface area. As a result, it is possible to efficiently capture (for example, adsorb) a specific substance in blood. In a case where the average particle diameter thereof is less than or equal to 10 µm, the effect becomes larger, and in a case where the average particle diameter thereof is less than or equal to 5 µm, the effect becomes even larger.

In the present specification, the "average particle diameter" means a particle diameter (also referred to as a median diameter D₅₀) at 50% of cumulative values in a particle size distribution obtained through a laser diffraction/light scattering method.

Animals from which blood to be purified by the blood purification device IE is derived are not particularly limited. The blood may be derived from humans or non-human animals such as livestock or pets. Examples of non-human animals include dogs, cats, monkeys, pigs, cows, horses, sheep, goats, mice, rats, and birds.

As described above, the blood purification device IE can extract a specific substance from blood to purify the blood. The blood purification device IE may be directly connected to an animal to immediately purify blood collected from the animal. Alternatively, the blood purification device IE can purify blood which has been collected from an animal in advance without being directly connected to the animal. The purified blood may be immediately returned to the individual animal or may be returned to the individual animal after being stored temporarily.

In the present embodiment, examples of specific substances in blood to be removed by magnetic particles include low-molecular-weight compounds, proteins, nucleic acids, and cells. More specific examples thereof include urea, creatinine, uric acid, β2-micro globulin, LDL cholesterol, abnormal antibodies in immune diseases, and substances, such as viruses, bacteria, fungi, and cancer cells, which cause diseases.

### (Sixth Embodiment)

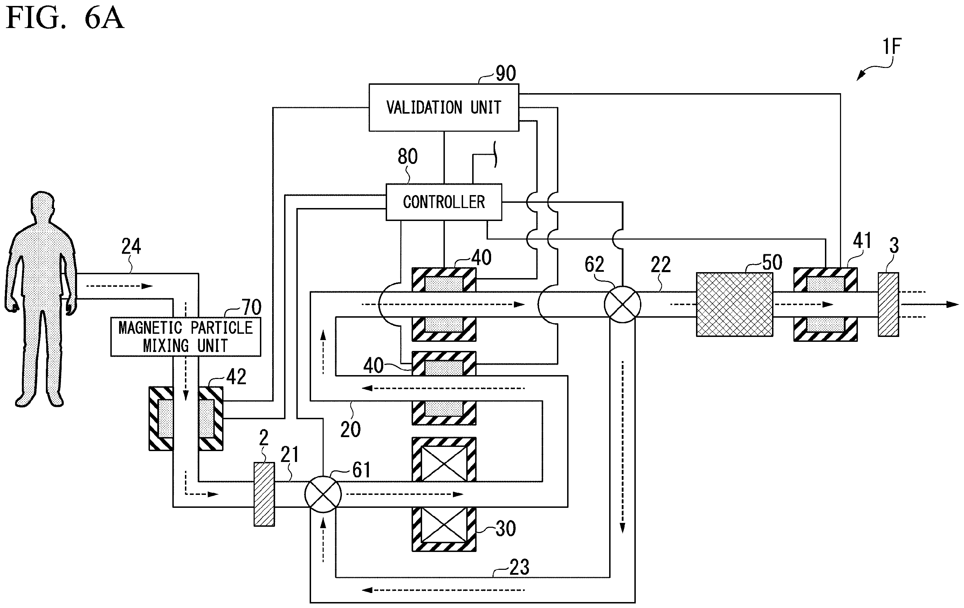

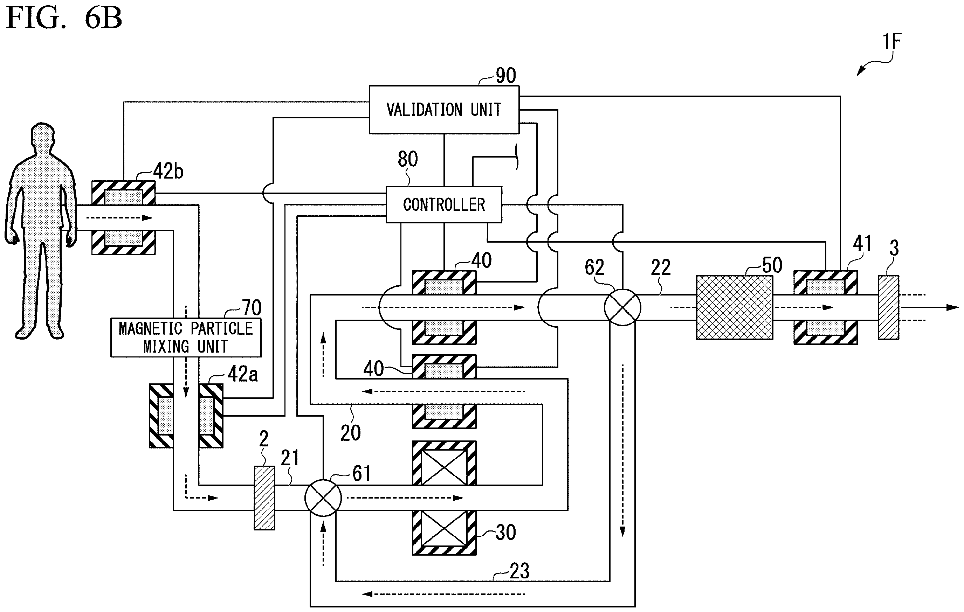

Figs. 6A and 6B are plan views showing configurations of a blood purification device IF according to a sixth embodiment of the present disclosure. In the present embodiment, a case where the blood purification device IF includes a meander-shaped return flow channel and a controller 80 for operating a flow channel selection unit will be described as an example.

As exemplified in Fig. 6A, the blood purification device IF includes: main flow channels 20, 21, and 22 through which blood 10 flows; a magnetic extraction unit 30 (magnetic extraction means) for collecting magnetic particles contained in the blood 10; and two magnetic sensors 40 and 40 and magnetic sensors 41 and 42 for detecting the presence of the magnetic particles in the blood 10; a magnetic particle mixing unit 70 for mixing the magnetic particles into the blood 10; and a controller 80 for processing measurement values of magnetic sensors. In addition, the blood purification device IF includes: a return flow channel 23 for recirculating the blood 10; a first flow channel selection unit 61 (first flow channel selection means) for selecting a flow channel; and a second flow channel selection unit 62 (second flow channel selection means) for selecting a flow channel. Furthermore, the blood purification device IF includes a filter 50, a connection port (inlet) 2, and a connection port (outlet) 3.

As exemplified in Fig. 6B, the blood purification device IF may include at least one of magnetic sensors 42a and 42b for detecting the presence of magnetic particles in the blood 10. Both the magnetic sensors 42a and 42b are exemplified in Fig. 6B as a specific example, but the blood purification device IF may include only the magnetic sensor 42b.

In the blood purification device IF, two magnetic sensors 40 and 40 are implemented in the main flow channel 20. The magnetic sensors 40 are disposed so as not to be affected by a magnetic field generated by the magnetic extraction unit 30. With such an arrangement, the magnetic sensors 40 can more accurately measure the magnetism of the blood 10.

In the blood purification device 1F, the two magnetic sensors 40 and 40 implemented in the main flow channel 20 are, for example, attached in a state of being deviated from the axis of the magnetic extraction unit 30 in a side view of the main flow channel 20. Furthermore, the two magnetic sensors 40 and 40 are also attached in a state in which their axes are deviated from each other. In this manner, the two magnetic sensors 40 and 40 are disposed so as not to be affected by a magnetic field generated by the magnetic extraction unit 30.

By providing the two magnetic sensors 40 in this blood purification device IF, it is possible to more accurately measure the magnetism of the blood 10 using two measurement values of the two magnetic sensors 40 and 40. In addition, with such an arrangement, malfunction hardly occurs in the magnetic sensors 40, and therefore, the measurement accuracy of the magnetic sensors 40 can be improved.

The magnetic particle mixing unit 70 is located on a preceding portion with respect to the magnetic extraction unit 30, and magnetic particles are mixed into the blood 10. However, the magnetic particle mixing unit 70 may be implemented in the blood purification device IF, or may be implemented outside the blood purification device IF.

The magnetic sensors 42, 42a, and 42b are implemented as parts preceding the magnetic extraction unit 30. The magnetic sensors 42 and 42a may be implemented on a subsequent portion with respect to the magnetic particle mixing unit 70. The magnetic sensor 42b may be located on a preceding portion with respect to the magnetic particle mixing unit 70.

The magnetic sensor 42 (42a) can measure magnetism indicated by blood after magnetic particles are mixed into the blood. In this case, a magnetism value measured by the magnetic sensor 42 (42a) may be used as a reference value (a "first reference value") after charging magnetic particles, for example.

The magnetic sensor 42b can measure magnetism of blood itself containing no magnetic particles before magnetic particles are mixed into the blood. In this case, a magnetism value measured by the magnetic sensor 42b may be used as a reference value (a "second reference value") before charging magnetic particles, for example.

The first flow channel selection unit 61 and the second flow channel selection unit 62 are, for example, three-way solenoid valves. Not limited to the above, the first flow channel selection unit 61 and the second flow channel selection unit 62 may be realized with an appropriate configuration (for example, an actuator or a valve) capable of selecting a flow channel of blood using control signals output from the controller 80, for example.

The magnetic extraction unit 30, the magnetic sensors 40 and 41 are disposed so as to come into contact with the main flow channel 20. The magnetic sensors 40 and 41 are implemented as parts subsequent to the magnetic extraction unit 30. In one embodiment of the blood purification device IF, the number of magnetic sensors implemented as parts subsequent to the magnetic extraction unit 30 may be three, however the number of magnetic sensor(s) may be one or two or may be four or more.

The controller 80 is connected to the two magnetic sensors 40 and 40, the magnetic sensors 41 and 42, the first flow channel selection unit 61, the second flow channel selection unit 62, and an artificial heart-lung machine (not shown in the drawings) (Fig. 6A). The controller 80 may be connected to the magnetic sensors 40, 41, 42a, and 42b, the first flow channel selection unit 61, the second flow channel selection unit 62, and an artificial heart-lung machine (not shown in the drawings) as exemplified in Fig. 6B. Signals, for example, from the two magnetic sensors 40 and 40 and the magnetic sensors 41 and 42 are input to the controller 80. Signals from the magnetic sensors 40, 41, 42a, and 42b may be input to the controller 80 (Fig. 6B). In addition, as an example, the controller 80 may output signals to the first flow channel selection unit 61, the second flow channel selection unit 62, and the artificial heart-lung machine. However, the controller 80 may not be directly connected to the magnetic sensor 41 and the artificial heart-lung machine. For example, the controller 80 may receive data of measurement values measured by the magnetic sensors 40, 41, 42, 42a, 42b, and the like as input.

First, the controller 80 outputs signals to the first flow channel selection unit 61 and operates the first flow channel selection unit 61 to allow communication between the main flow channel 21 and the main flow channel 20. In addition, the controller 80 outputs signals to the second flow channel selection unit 62 and operates the second flow channel selection unit 62 to allow communication between the main flow channel 20 and the return flow channel 23 and block communication between the main flow channels 20 and 22. Accordingly, the blood 10 containing magnetic particles flows from the connection port 2 into the main flow channel 21.

After the blood 10 containing magnetic particles flows in from the connection port 2, the controller 80 operates the first flow channel selection unit 61 to allow communication between the main flow channel 20 and the return flow channel 23. At this time, the second flow channel selection unit 62 allows a state where the main flow channel 20 is allowed to communicate with the return flow channel 23 to continue.

The blood 10 circulates in the main flow channel 20 and the return flow channel 23. During that period, the magnetic particles are collected by the magnetic extraction unit 30, and the magnetism generated from the blood 10 is measured by the magnetic sensors 40 and 40.

As one specific example, the controller 80 may execute processing of comparing the magnetism of the blood measured by the magnetic sensors 40 and 40 with the magnetism (first reference value) which has been measured by the magnetic sensor 42 (42a) and is indicated by the blood containing magnetic particles. In this case, the controller 80 can, for example, output the results of the processing to an appropriate display device or the like. In addition, the controller 80 may control at least one of the first flow channel selection unit 61 and the second flow channel selection unit 62 according to the results obtained by comparing the magnetism of the blood measured by the magnetic sensors 40 and 40 with the first reference value measured by the magnetic sensor 42 (42a).

In a case where, for example, the controller 80 determines that either or both of the magnetism values of the blood measured by the two magnetic sensors 40 and 40 are larger than the first reference value measured by the magnetic sensor 42 (42a), the controller may control the first flow channel selection unit 61 and the second flow channel selection unit 62 to allow the state where the main flow channel 20 is allowed to communicate with the return flow channel 23 to continue. In addition, in a case where, for example, the controller 80 determines that the difference between the first reference value measured by the magnetic sensor 42 (42a) and either or both of the magnetism values of the blood measured by the two magnetic sensors 40 and 40 is not included in a range of specific reference values (for example, in a case where the difference therebetween is smaller than a certain standard), the controller may control the first flow channel selection unit 61 and the second flow channel selection unit 62 to allow the state where the main flow channel 20 is allowed to communicate with the return flow channel 23 to continue.

As another specific example, the controller 80 may execute processing of comparing the magnetism of the blood measured by the magnetic sensors 40 and 40 with the magnetism (second reference value) which has been measured by the magnetic sensor 42b and is indicated by the blood containing no magnetic particles. In addition, the controller 80 may control at least one of the first flow channel selection unit 61 and the second flow channel selection unit 62 according to the results obtained by comparing the second reference value with the magnetism of the blood measured by the magnetic sensors 40 and 40.

Specifically, in a case where, for example, the controller 80 determines that either or both of the magnetism values of the blood measured by the two magnetic sensors 40 and 40 are larger than the second reference value measured by the magnetic sensor 42b, the controller may control the first flow channel selection unit 61 and the second flow channel selection unit 62 to allow the state where the main flow channel 20 is allowed to communicate with the return flow channel 23 to continue. In a case where, for example, the controller 80 determines that the difference between the second reference value measured by the magnetic sensor 42b and either or both of the magnetism values of the blood measured by the two magnetic sensors 40 and 40 is not included in a range of specific reference values (for example, in a case where the difference therebetween is larger than a certain standard), the controller may control the first flow channel selection unit 61 and the second flow channel selection unit 62 to allow the state where the main flow channel 20 is allowed to communicate with the return flow channel 23 to continue.

On the other hand, when the controller 80 determines that the blood 10 flowing through the main flow channel 20 contains no magnetic particles (or that the amount of magnetic particles is sufficiently reduced) based on the magnetism of the blood measured by either or both of the two magnetic sensors 40 and 40 and the first reference value measured by the magnetic sensor 42 (42a), the controller may execute the following processing. That is, the controller 80 outputs signals to the second flow channel selection unit 62 and operates the second flow channel selection unit 62 to allow communication between the main flow channel 20 and the main flow channel 22, for example. At this time, the controller 80 may control the first flow channel selection unit 61 so as to allow the state where the main flow channel 20 is allowed to communicate with the return flow channel 23 to continue. Then, the blood 10 flows into the main flow channel 22 and passes through the filter 50. As an example, in a case where the difference between the first reference value measured by the magnetic sensor 42 (42a) and either or both of the magnetism values of the blood measured by the two magnetic sensors 40 and 40 is included in a specific range (for example, in a case where the difference therebetween is larger than a certain standard), the controller 80 may operate the second flow channel selection unit 62 to allow communication between the main flow channel 20 and the main flow channel 22.

In addition, when the controller 80 determines that the blood 10 flowing through the main flow channel 20 contains no magnetic particles (or that the amount of magnetic particles is sufficiently reduced) based on the magnetism of the blood measured by either or both of the two magnetic sensors 40 and 40 and the second reference value measured by the magnetic sensor 42b, the controller may execute the following processing. That is, the controller 80 outputs signals to the second flow channel selection unit 62 and operates the second flow channel selection unit 62 to allow communication between the main flow channel 20 and the main flow channel 22, for example. At this time, the controller 80 may control the first flow channel selection unit 61 so as to allow the state where the main flow channel 20 is allowed to communicate with the return flow channel 23 to continue. Then, the blood 10 flows into the main flow channel 22 and passes through the filter 50. As an example, in a case where the difference between the second reference value measured by the magnetic sensor 42b and either or both of the magnetism values of the blood measured by the two magnetic sensors 40 and 40 is included in a specific range (for example, in a case where the difference therebetween is smaller than a certain standard), the controller 80 may operate the second flow channel selection unit 62 to allow communication between the main flow channel 20 and the main flow channel 22.

In addition, the controller 80 can determine whether or not the blood 10 flowing through the main flow channel 22 contains magnetic particles (or whether or not the amount of magnetic particles is sufficiently reduced) based on the magnetism of the blood measured by the magnetic sensor 41 and the magnetism (first reference value) which has been measured by the magnetic sensor 42 (42a) and is indicated by blood itself containing magnetic particles. In addition, the controller 80 may determine whether or not the blood 10 flowing through the main flow channel 22 contains magnetic particles (or whether or not the amount of magnetic particles is sufficiently reduced) based on the magnetism of the blood measured by the magnetic sensor 41 and the magnetism which has been measured by the magnetic sensor 42b and is indicated by blood itself containing no magnetic particles.

Specifically, in a case where, for example, the magnetism of the blood measured by the magnetic sensor 41 is larger than the first reference value, the controller 80 may determine that the amount of magnetic particles is not sufficiently reduced. In addition, in a case where, for example, the difference between the first reference value and the magnetism of the blood measured by the magnetic sensor 41 is not included in a range of specific reference values (for example, in a case where the difference therebetween is smaller than a reference value), the controller 80 may determine that the amount of magnetic particles is not sufficiently reduced.

Similarly, in a case where, for example, the magnetism of the blood measured by the magnetic sensor 41 is larger than the second reference value, the controller 80 may determine that the amount of magnetic particles is not sufficiently reduced. In addition, in a case where, for example, the difference between the second reference value and the magnetism of the blood measured by the magnetic sensor 41 is not included in a range of specific reference values (for example, in a case where the difference therebetween is larger than a reference value), the controller 80 may determine that the amount of magnetic particles is not sufficiently reduced.

The controller 80 can compare the first reference value measured by the magnetic sensor 42 (42a) with the magnetism of the blood measured by the magnetic sensor 40 to output the results to a display device, for example. The controller 80 can compare the second reference value measured by the magnetic sensor 42b with the magnetism of the blood measured by the magnetic sensor 40 to output the results to a display device, for example.

In a case where the controller 80 determines that the amount of magnetic particles is not sufficiently reduced based on the first reference value measured by the magnetic sensor 42 (42a) and the magnetism of the blood measured by the magnetic sensor 41, the controller may output signals for controlling the operation of the artificial heart-lung machine. Similarly, in a case where the controller 80 determines that the amount of magnetic particles is not sufficiently reduced based on the second reference value measured by the magnetic sensor 42b and the magnetism of the blood measured by the magnetic sensor 41, the controller may output signals for controlling the operation of the artificial heart-lung machine. In this case, the controller 80 may control, for example, the artificial heart-lung machine so as to stop the blood 10 from flowing into the artificial heart-lung machine. At this time, the controller 80 may control at least one of the first flow channel selection unit 61 and the second flow channel selection unit 62 to block communication between the main flow channel 22 and the return flow channel 23 and to switch the flow channels into a state where the main flow channel 20 is allowed to communicate with the return flow channel 23. Accordingly, the controller 80 can prevent the blood 10, which contains magnetic particles, from flowing into the artificial heart-lung machine.

In this blood purification device 1F, the controller 80 operates the first flow channel selection unit 61 and the second flow channel selection unit 62 based on the measurement values of the two magnetic sensors 40 and 40 and the magnetic sensors 42 (42a) and 42b. Therefore, the measurement accuracy of the magnetic sensors 40 can be improved and magnetic particles can be sufficiently separated and removed from blood before purified blood is returned to the body.

The filter 50 can capture, for example, the magnetic particles, which could not be removed by the magnetic extraction unit 30, and foreign substances contained in the blood 10.

### <Magnetic Extraction Unit>

The magnetic extraction unit 30 can collect magnetic particles in blood flowing through the main flow channel 20 with a magnetic force. Accordingly, a specific substance captured by the magnetic particles can be extracted from blood.

Known methods can be employed to implement the magnetic extraction unit 30. For example, magnetic separation method disclosed in Patent Literature 1 can be used.

The collection of magnetic particles from blood may be performed, for example, by taking out the magnetic particles into a flow channel connected to the main flow channel 20, or magnetic particles may be collected in a collection unit located on an inner wall of the main flow channel 20.

The magnetic extraction unit 30 may have magnetic field generator for generating a magnetic field. Magnetic particles are collected from the blood 10 flowing through the main flow channel 20 due to this magnetic field. The magnetic field generator may be, for example, a permanent magnet or a variable magnetic flux magnet, or may be, for example, an induced magnetic field.

The magnetic extraction unit 30 may have a member, such as a magnetic shield layer, capable of shielding a magnetic field. Accordingly, it is possible to suppress or prevent a leakage magnetic field from the magnetic extraction unit 30.

The magnetic field generator may be disposed outside the main flow channel 20 and free of contact with the blood 10. Since the magnetic field generator does not come into contact with the blood 10, coagulation of the blood 10 and contamination with foreign substances can be suppressed.

### <Magnetic Sensor>

The magnetic sensors 40, 41, 42 (42a), and 42b can detect the presence of magnetic particles by detecting a leakage magnetic field generated from magnetic particles in blood flowing through the main flow channel 20.

The magnetic sensors 40, 41, 42 (42a), and 42b may be disposed outside the main flow channel 20 and free of contact with the blood 10. Since the magnetic sensors 40, 41, 42 (42a), and 42b are free of contact with the blood 10, coagulation of the blood 10 and contamination with foreign substances can be suppressed.

Known magnetic sensors can be used as the magnetic sensors 40, 41, 42 (42a), and 42b. For example, coil-type sensors may be used, Hall sensors may be used, or elements using magnetic resistance may be used. It is possible to determine whether or not the blood 10 flowing through the main flow channel 20 contains magnetic particles based on measurement values of magnetism indicated by the magnetic sensors 40, 41, 42 (42a), and 42b. The magnetic sensors 40, 41, 42 (42a), and 42b may be the same or different types of magnetic sensors.

A coil-type sensor measures and detects a magnetic field of magnetic particles which are passing through, using an induction coil when a magnetic particle-containing sample is passed through the induction coil. The coil-type sensor has an advantage of being able to be easily installed.

A Hall sensor detects a magnetic field using the Hall effect and has advantages of being inexpensive and able to be easily installed.

A magnetoresistive element is an element utilizing a phenomenon in which electrical resistance changes due to an effect of a magnetic field, and includes an anisotropic magnetoresistive effect (AMR) element, a giant magnetoresistive (GMR) element, a tunnel magnetoresistive resistance (TMR) element, and the like. A GMR element or a TMR element has advantages that the temperature change or the change with time is small and the sensitivity (MR ratio) is large.

The magnetic sensors 40, 41, 42 (42a), and 42b may have a member, such as a magnetic shield layer, capable of shielding a magnetic field. Accordingly, it is possible to prevent failures such as false detection due to influence of a magnetic field generated from the magnetic extraction unit 30.

### <Blood Pump>

The blood purification device IF may be connected to a blood pump capable of making blood flow. The blood pump may be, for example, used for taking out blood from the body or used for circulating blood taken out of the body. Alternatively, an externally provided blood pump may be used separately from the blood purification device of the present disclosure.

In this blood purification device 1F, for example, the blood 10 containing magnetic particles modified with the separation-target capturing material, which is capable of capturing a specific substance in the blood, is allowed to flow through the main flow channels 20, 21, and 22 through which the blood 10 can flow. In addition, the magnetic extraction unit 30 capable of generating a magnetic field collects at least the portion of the magnetic particles contained in the blood 10. The magnetic sensors 40 and 41 capable of detecting the presence of the magnetic particles detect whether or not the magnetic particles have remained in the blood after at least the portion of the magnetic particles are collected by the magnetic extraction unit 30.

Although the sixth embodiment of the present disclosure has been described in detail above, the technology according to the present disclosure is not limited to the sixth embodiment and various modifications and changes can be made within the scope of the gist of the present disclosure disclosed in the claims.

For example, the temperature of the entire blood purification device IF in the sixth embodiment may be kept constant or within a specific temperature range. For example, an exterior cover which externally covers the entire blood purification device and is composed of a heat insulating material or the like may be provided in the blood purification device. The measurement values of the magnetic sensors are stabilized by keeping the temperature of the entire device constant. In addition, the temperature of the entire blood purification device is preferably kept the same as body temperature. For example, the blood purification device may further include: a temperature sensor (not shown in the drawings) that detects the internal temperature of the above-described exterior cover; and a heating unit (not shown in the drawings) of a heater or the like that heats the interior of the above-described exterior cover according to signals from the controller based on the temperature sensor. Accordingly, it is possible to stabilize the measurement values of the magnetic sensors and to maintain the quality of blood flowing through the blood purification device for a long period of time.

In addition, as exemplified in Figs. 6A and 6B, the blood purification device IF may further include a validation unit 90 which determines whether or not the magnetic sensors 40, 40, 41 42 (42a), and 42b are operating correctly. For example, the validation unit 90 may be connected to the magnetic sensors 40, 40, 41, 42 (42a), and 42b and determine whether the magnetic sensors 40, 40, 41, 42 (42a), and 42b are operating correctly on a basis of the outputs (magnetic values of blood) from the magnetic sensors. The validation unit may be configured in the same manner as the validation unit 90 of the above-described first embodiment, for example.

In addition, the blood purification device IF may further include a controller which is connected to the magnetic sensors 40, 40, 41, 42 (42a), and 42b and determines whether or not magnetic particles are contained in the blood 10 on a basis of the outputs from the magnetic sensors. The controller 80 may check operation states of the magnetic sensors 40, 40, 41, 42 (42a), and 42b which have been determined by the validation unit 90. Accordingly, the controller 80 which is a safety circuit can check the operations of the magnetic sensors 40, 40, 41, 42 (42a), and 42b. As a result, the controller can accurately determine whether or not magnetic particles are contained in the blood 10.

### <Magnetic Particles>

Examples of magnetic particles include particles exhibiting ferromagnetism or paramagnetism. At least a part of the outer circumferential portion of each magnetic particle is modified with a separation-target capturing material which is capable of capturing a specific substance in blood.

The magnetic particles may include: for example, iron, cobalt, nickel, and inorganic compounds thereof; and organically modified inorganic compounds obtained so that these metals or inorganic compounds are modified with organic compounds.

The magnetic particles can capture a specific substance in blood through the separation-target capturing material. A method of capturing a specific component in blood by the separation-target capturing material is not particularly limited. For example, a chemical bonding, adsorption, and trapping using a three-dimensional structure can be appropriately adopted. Magnetic particles may be coated with coating materials including the separation-target capturing material. Also, whole body magnetic particle may be formed from the separation-target capturing material itself. In addition, magnetic particles may be embedded in the separation-target capturing material being formed in a granular shape.

At least a part of the outer circumferential portion of a magnetic particle is preferably coated with a polymer or a silica matrix depending on the component to be separated out.

At least a part of the outer circumferential portion of a magnetic particle may have hydrophobicity or hydrophilicity depending on the component to be separated out.

The average particle diameter of magnetic particles may be greater than or equal to 2 nm. In a case where the average particle diameter of magnetic particles is within the above-described range, the magnetic particles can be efficiently collected by the magnetic field generator included in the magnetic extraction unit 30. In a case where the average particle diameter thereof is greater than or equal to 100 nm, the effect becomes larger, and in a case where the average particle diameter thereof is greater than or equal to 250 nm, the effect becomes even larger.

The average particle diameter of magnetic particles may be less than or equal to 1 mm. In a case where the average particle diameter of magnetic particles is within the above-described range, the magnetic particles have high dispersibility and a large specific surface area. As a result, it is possible to efficiently capture (for example, adsorb) a specific substance in blood. In a case where the average particle diameter thereof is less than or equal to 10 µm, the effect becomes larger, and in a case where the average particle diameter thereof is less than or equal to 5 µm, the effect becomes even larger.

In the present specification, the "average particle diameter" means a particle diameter (also referred to as a median diameter D₅₀) at 50% of cumulative values in a particle size distribution obtained through a laser diffraction/light scattering method.

Animals from which blood to be purified by the blood purification device IF is derived are not particularly limited. The blood may be derived from humans or non-human animals such as livestock or pets. Examples of non-human animals include dogs, cats, monkeys, pigs, cows, horses, sheep, goats, mice, rats, and birds.

As described above, the blood purification device IF can extract a specific substance from blood to purify the blood. The blood purification device IF may be directly connected to an animal to immediately purify blood collected from the animal. Alternatively, the blood purification device IF can purify blood which has been collected from an animal in advance without being directly connected to the animal. The purified blood may be immediately returned to the individual animal or may be returned to the individual animal after being stored temporarily.

In the present embodiment, examples of specific substances in blood to be removed by magnetic particles include low-molecular-weight compounds, proteins, nucleic acids, and cells. More specific examples thereof include urea, creatinine, uric acid, β2-micro globulin, LDL cholesterol, abnormal antibodies in immune diseases, and substances, such as viruses, bacteria, fungi, and cancer cells, which cause diseases.

As described above, the technology according to the present disclosure has been described in detail with reference to the above-described embodiments. The technology according to the present disclosure is not limited to the above-described embodiments. That is, for example, modified embodiments in which at least a part of each of the above-described embodiments is modified, changed, or improved within a range of technical ideas according to the present disclosure described in the claims, a combination of one or more of the embodiments, and a combination of each of the above-described embodiments and an improved embodiment can be included in the technology according to the present disclosure.

### [Reference Signs List]

- 1A: Blood purification device
- 1B: Blood purification device
- 1C: Blood purification device
- 1D: Blood purification device
- 1E: Blood purification device
- IF: Blood purification device
- 2: Connection port (inlet)
- 3: Connection port (outlet)
- 10: Blood
- 20: Main flow channel
- 21: Main flow channel
- 22: Main flow channel
- 23: Return flow channel
- 30: Magnetic extraction unit (magnetic extraction means)
- 40: Magnetic sensor
- 41: Magnetic sensor
- 42: Magnetic sensor
- 42a: Magnetic sensor
- 42b: Magnetic sensor
- 50: Filter
- 61: First flow channel selection unit (first flow channel selection means)
- 62: Second flow channel selection unit (second flow channel selection means)
- 70: Magnetic particle mixing unit
- 80: Controller
- 90: Validation unit

## Claims

1. A blood purification device comprising:
a main flow channel configured to allow blood to flow through;
a magnetic extraction means configured to collect magnetic particles with a magnetic force, the magnetic particles being contained in blood; and
one or more magnetic sensors configured to be capable of detecting a presence of the magnetic particles in blood,
wherein each of the magnetic particles has, on at least a part of its outer circumferential portion, a modified part being modified with a separation-target capturing material which is capable of capturing a specific substance to be separated in the blood,
and wherein the magnetic extraction means is further configured to collect the magnetic particles that have captured the specific substance and,to extract the collected specific substance from the blood thereby purifying the blood.

2. The blood purification device according to claim 1,
wherein the at least one magnetic sensor among the one or more magnetic sensors is disposed on a subsequent portion with respect to the magnetic extraction means.

3. The blood purification device according to claim 2,
wherein another at least one magnetic sensor among the one or more magnetic sensors is disposed on a preceding portion with respect to the magnetic extraction means.

4. The blood purification device according to any one of claims 1 to 3, further comprising:
a first flow channel selection means disposed on a preceding portion with respect to the magnetic extraction means in the main flow channel and configured to select a flow destination of the blood;
a second flow channel selection means disposed on a subsequent portion with respect to the magnetic extraction means in the main flow channel and configured to select a flow destination of the blood; and
a return flow channel disposed between the first flow channel selection means and the second flow channel selection means.

5. The blood purification device according to claim 4, further comprising:
a magnetic sensor disposed on a subsequent portion with respect to the second flow channel selection means in the main flow channel.

6. The blood purification device according to claim 4 or 5, further comprising:
a controller connected to the magnetic sensors, the first flow channel selection means, and the second flow channel selection means,
wherein the controller is configured to control at least one of the first flow channel selection means and the second flow channel selection means on a basis of outputs of the magnetic sensors.

7. The blood purification device according to any one of claims 1 to 6,
wherein the magnetic sensors are magnetoresistive elements which are disposed outside the flow channel so as not to contact with the blood.

8. The blood purification device according to any one of claims 1 to 6,
wherein the magnetic sensors are coil-type sensors which are disposed outside the flow channel so as not to contact with the blood.

9. The blood purification device according to any one of claims 1 to 6,
wherein the magnetic sensors are Hall sensors which are disposed outside the flow channel so as not to contact with the blood.

10. The blood purification device according to any one of claims 1 to 9,
wherein the magnetic extraction means comprises a magnetic field generator configured to generate a magnetic field for collecting the magnetic particles, and
wherein the magnetic field generator is disposed at a position outside the flow channel so as not to contact with the blood.

11. The blood purification device according to any one of claims 1 to 10,
wherein a temperature of the blood purification device itself is kept constant or within a specific temperature range.

12. The blood purification device according to any one of claims 1 to 11, further comprising:
a validation unit which determines whether the magnetic sensors are normally operational.

13. A purification method of blood comprising the steps of:
flowing blood containing magnetic particles through a main flow channel being configured to allow the blood to flow through, each of the magnetic particles being modified with a separation-target capturing material which is capable of capturing a specific substance to be separated in blood;
collecting at least a portion of the magnetic particles contained in the blood using magnetic extraction means capable of generating a magnetic field; and
detecting, by a magnetic sensor, remaining of the magnetic particles in the blood after the at least the portion of the magnetic particles being collected by the magnetic extraction means, the magnetic sensor capable of detecting the presence of the magnetic particles.
